# EUROPEAN PATENT SPECIFICATION

(11) **EP 4 032 530 B1**
(45) Date of publication and mention of the grant of the patent: **09.04.2025**
(21) Application number: 21153203.1
(22) Date of filing: 25.01.2021
(51) Int. Cl.: A61K 9/24, A61K 31/4178, A61P 31/04

(54) **PROLONGED-RELEASE FURAZIDIN COMPOSITION**
FURAZIDIN-ZUSAMMENSETZUNG MIT VERLÄNGERTER FREISETZUNG
COMPOSITION DE FURAZIDINE À LIBÉRATION PROLONGÉE

(43) Date of publication of application: 27.07.2022
(73) Proprietor: Adamed Pharma S.A., 05-152 Czosnow (PL)
(72) Inventor: Trela, Jolanta, 95-200 Pabianice (PL); Kubiak, Bartlomiej, 05-101 Nowy Dwor Mazowiecki (PL); Kiryluk, Anna, 05-800 Pruszkow (PL); Niemczyk, Katarzyna, 32-600 Oswiecim (PL)
(74) Representative: Hoffmann Eitle

(56) References cited:
- EP-A1- 3 643 325
- KIRTI D TIRKHUNDE ET AL: "FORMULATION AND EVALUATION OF NITROFURANTOIN BI-LAYER TABLET", WORLD JOURNAL OF PHARMACEUTICAL RESEARCH, vol. 7, no. 18, November 2018 (2018-11-01), pages 1277 - 1290, XP055630287, ISSN: 2277-7105, Retrieved from the Internet <URL:https://wjpr.net/download/article/1541036788.pdf> [retrieved on 20191009], DOI: 10.20959/wjpr201818-13648

## Description

### Field of the present invention

The present invention relates to a prolonged-release pharmaceutical composition of furazidin for oral administration, which can be used for treating urinary tract infections (UTI).

### Technological background

Urinary tract infections (UTI) are the most common condition requiring medical consultation and care in outpatients. UTIs account for approx. 10-20% of all community-acquired infections and for approx. 40-50% of nosocomial infections. Approx. 80% of all UTIs occur in women. According to epidemiological studies, bacteriuria occurs at least once in a lifetime of approx. 10% of female population and in 1-2% of male population, with asymptomatic UTIs in women probably occurring twice as frequently.

An uncomplicated urinary tract infection (uUTI) is a clinical syndrome characterized by pyuria and a documented microbial pathogen on urine culture, accompanied by local signs and symptoms such as urinary frequency, urinary urgency, dysuria, and suprapubic pain. Uncomplicated UTIs (uUTI), often also referred to as acute cystitis, occur in females with normal anatomy of the urinary tract and are not accompanied by systemic signs or symptoms, such as fever higher than 38 degrees Celsius or costovertebral angle pain. Urinary tract infections in males are characterized as complicated UTI (cUTI) because these infections occur in association with urologic abnormalities such as instrumentation or bladder outlet obstruction (e.g., benign prostatic hypertrophy).

A UTI is classified as uncomplicated if there are no relevant functional or anatomical anomalies in the urinary tract, no relevant renal functional impairment and no relevant concomitant disease that would promote the UTI, or the risk of developing serious complications. Examples of uncomplicated UTI include:
- Acute uncomplicated cystitis (AUC): A lower UTI (cystitis) is assumed when the acute symptoms refer only to the lower urinary tract, for example, urgency, pain on micturition (dysuria), frequency, and pain above the symphysis.
- Acute uncomplicated pyelonephritis: An upper UTI (pyelonephritis) ought to be assumed if, in the case of acute symptoms, flank pain, pain on palpation of the flank, and/or fever (>38°C) persist.
- Asymptomatic bacteriuria: Clinically symptomatic UTI is distinguished from asymptomatic bacteriuria. The term 'asymptomatic UTI' ought not to be used.
- Recurrent uncomplicated UTIs: A recurrent UTI is assumed if relapse rates of ≥2 symptomatic episodes occur within 6 months or ≥3 symptomatic episodes within 12 months.

UTIs are a significant clinical problem due to their serious sequelae, such as septic conditions, urolithiasis, renal failure, hypertension or occurrence of these complications during pregnancy. Community-acquired infections most often occur as a result of ascending invasion of native bacterial flora coming from the gastrointestinal tract. In typical cases, the first stage of infection is colonisation of the urinary meatus (in males; in females, the vestibule of the vagina), followed by urinary bladder invasion through the urethra. In women, because of the close proximity of the vagina, rectum and urinary meatus, shortness of the urethra as well as mechanical introduction of microorganisms into the urethra during sexual intercourse, the risk of infections is higher than in men.

Recurrent UTIs are defined as infections characterised by at least 3 episodes within one year or 2 episodes within 6 months. Infection recurrence is understood as a repeated UTI episode caused by the same micro-organism, i.e. by its endospores, within 3 weeks of the end of treatment. Such recurrences are often asymptomatic. Reinfections are repeated infections occurring after the urinary tract has been sterilised using antibacterial agents, but caused by a different bacterial strain, occurring up to 14 days after sterilisation of urine. In the population of young, healthy and sexually active women, 10% experience at least one UTI episode a year, and the risk of recurrent UTIs is approx. 0.5- 0.7/person/year.

Despite of the severe course and the recurrent nature of UTI, its treatment is still initiated empirically (without bacteriological assessment of urine). This means that, in a considerable proportion of patients treated with quinolines or trimethoprim/sulfamethoxazole (being a standard), this treatment might be unsuccessful due to increased antibiotic resistance to these products that has been witnessed in the recent years. In view of the epidemic of UTIs and the increasing drug resistance of bacterial strains, a need arises for an efficacious drug characterised by favourable safety, efficacy and patient acceptance profile.

Despite the growing microbial resistance, furazidin is efficient in the treatment of different forms of UTI. It is also safe, exhibiting less side effects, comparing to other antimicrobial agents and may be administered to children as well. The efficacy results from the high sensitivity of micro-organisms to furazidin. Moreover, the low resistance of uropathogens to furazidin is virtually unchanging in time, contrary to what is observed in the case of other antibiotics or chemotherapeutic agents. *Escherichia coli,* the most frequent pathogen causing acute UTIs (70-95% of cases), is susceptible to furazidin at a constant level of approx. 90%, which has not changed for several decades, with simultaneous increase observed in drug resistance to quinolines or trimethoprim/sulfamethoxazole and fosfomycin. The unchanging and high susceptibility of microbial strains to furazidin makes it a very suitable candidate for the first-choice oral drug in empiric therapy of UTIs.

However, upon oral administration, furazidin has a very short half-life (below 1 hour). Blood drug concentrations of furazidin decrease very quickly after administration, reaching levels lower than the minimum concentration for exhibiting therapeutic effects. This creates a necessity of frequent administration of furazidin, which is according to the prior art administered in the form of immediate release (IR) tablets, containing 50 or 100 mg of furazidin. Due to the unfavourable pharmacokinetics, furazidin must be administered 3 to 4 times daily (each time 100 mg or 2 x 50 mg of furazidin) in order to maintain an effective concentration of furazidin in the plasma. Patient's compliance is however difficult because patients are reluctant to take furazidin in constant intervals 3 or 4 times a day (up to 8 tablets), in particular, as furazidin should be taken with a meal. Thus, eating times need to be adjusted to the dosage regimen. Even under the assumption of patients' complete adherence to the dosing schedule, it is very difficult to maintain therapeutic concentrations plasma and urine throughout the day.

Another problem associated with the conventional furazidin treatment is the "first pass effect". Upon oral administration, furazidin is quickly absorbed in the gastrointestinal tract, reaching high blood concentrations. During this time, intensive liver metabolism occurs, causing exacerbation of adverse effects.

Furthermore, EP 3 643 325 A1 is concerned with a pharmaceutical oral composition comprising furazidin in the form of powder, granules or minitablets, which comprises furazidin and at least one bulking agent chosen from the group consisting of starch, gelatinized starch, microcrystalline cellulose, lactose, glucose, mannitol, sorbitol, anhydrous colloidal silica, talc, dextrins or/and their mixture. The composition may be directly administered to a patient, it may be used for preparation of an oral suspension or a filling of a capsule.

Tirkhunde et al. (World Journal of Pharmaceutical Research, 2018, Vol. 7, pages 1277-1290) report on a nitrofurantoin bilayer tablet, which comprises an immediate release layer and a sustained-release layer. The sustained-release layer contains Carbopol71G as the control releasing agent.

### Summary of the invention

It is an object of the present invention to provide a pharmaceutical composition of furazidin that reduces or overcomes at least some of the above-mentioned problems.

The object is solved by the prolonged-release pharmaceutical composition and the pharmaceutical composition for use according to the appended claims.

In particular, the present invention relates to the following aspects:
(1) According to an aspect of the present invention, a prolonged-release pharmaceutical composition for oral administration as defined in the appended claims is provided.
(2) In the pharmaceutical composition, furazidin may be contained as the free base or as a pharmaceutically acceptable salt thereof, preferably as the free base.
(3) The tablet may be uncoated or coated. Preferably, the tablet is uncoated.
(4) According to an embodiment of the pharmaceutical composition, the immediate release component a) is contained as granulates, and the modified-release component b) is contained as granulates. Optionally, the immediate release component a) and/or the modified-release component b) also contain an extragranular fraction, for example, comprising one or more of a lubricant, a filler, an absorbent and a glidant.
(5) The pharmaceutical composition can comprise, in total, 150 to 250 mg of furazidin, preferably 180 to 220 mg of furazidin, and more preferably 200 mg of furazidin.
(6) If the pharmaceutical composition is according to (5), the immediate release component a) can comprise 35 to 55 mg of furazidin, and the modified-release component b) can comprise 145 to 165 mg of furazidin; and, preferably, the immediate release component a) comprises 45 to 55 mg of furazidin, and the modified-release component b) comprises 145 to 155 mg of furazidin; and, more preferably, the immediate release component a) comprises 50 mg of furazidin, and the modified-release component b) comprises 150 mg of furazidin.
(7) If the pharmaceutical composition is according to (5) or (6), the immediate release component a) has, in particular, a furazidin content of 32 to 50 wt.%, preferably 41 to 50 wt.%, more preferably 45 to 46 wt.%, such as 45.45 wt.%, based on the total weight of the immediate release component a); furthermore, the modified release component b) has, in particular, a furazidin content of 40 to 60 wt.%, preferably 52 to 56 wt.%, more preferably 54 to 55 wt.%, such as 53.57 wt.%, based on the total weight of the modified-release component b).
(8) In the pharmaceutical composition, the modified-release component b) may comprise 10 to 40 wt.% of the controlled-release agent, preferably 10 to 35 wt.% and more preferably 20 to 30 wt.% of the controlled-release agent, based on the total weight of the modified-release component b).
(9) In a matrix of the modified-release component b), the controlled-release agent comprises at least one pH-independent polymer. Preferably, the matrix is formed by the pH-independent polymer, or by the pH-independent polymer and a binder. More preferably, a thickness ratio of the thickness of the matrix of the pH-independent polymer in a swollen state to the thickness of the dry matrix of the pH-independent polymer before swelling is in the range of 1.1 to 1.8, preferably 1.3 to 1.6, wherein the swollen state is obtained by immersing the dry modified release component b) in 30 to 35 mL of phosphate buffer of pH=4.5 contained in beaker for 2 h, the beaker being placed in a wiggled, heated bath at 37°C.
(10) It is preferred that the pH-independent polymer has a weight average molecular weight of 50,000 to 7,000,000, preferably 350,000 to 800,000. Preferred examples of hydroxypropylmethylcellulose (HPMC) have a weight average molecular weight (Mw) of 70,000 to 1,000,000, preferably 70,000 to 600,000, more preferably 70,000 to 170,000. Preferred examples of carboxymethylcellulose (CMC) have a weight average molecular weight of 200,000 to 500,000, preferably 350,000 to 450,000, more preferably 370,000 to 400,000. Preferred examples of hydroxyethylcellulose (HEC) have a weight average molecular weight of 50,000 to 200,000, preferably 80,000 to 150,000, more preferably 80,000 to 100,000.
(11) It is preferred that the pH-independent polymer is
   - a combination of hydroxypropylmethylcellulose K4M and hydroxyethylcellulose,
   - a combination of hydroxypropylmethylcellulose K4M and non-ionic poly(ethylene oxide),
   - a combination of hydroxypropylmethylcellulose K4M and hydroxypropylmethylcellulose K100LV (Mw = 160,000 to 170,000),
   - a combination of hydroxypropylmethylcellulose K4M and hydroxypropylcellulose,
   - a combination of hydroxypropylmethylcellulose K15M and xanthan gum, or
   - a combination of hydroxypropylmethylcellulose K15M and sodium carboxymethylcellulose. Preferably, the pH-independent polymer is a combination of hydroxypropylmethylcellulose K4M and hydroxyethylcellulose.
(12) The immediate release component a) may comprise, as the at least one excipient, at least one of a filler, a disintegrant, a binder, a lubricant, a glidant and any combination thereof. Preferably the immediate release component a) comprises a filler, a disintegrant, a glidant, a lubricant, and, optionally, a binder. More preferably, the immediate release component a) contains a binder in addition to the filler, the disintegrant, the glidant and the lubricant.
(13) The modified-release component b) may comprise, as the at least one excipient, at least one of a filler, a disintegrant, a binder, a lubricant, a glidant and any combination thereof. Preferably, the modified-release component b) comprises a filler, a disintegrant, a binder, a lubricant, and, optionally, a glidant. More preferably, the modified-release component b) contains no glidant.
(14) The immediate release component a) and/or the prolonged-release component b) may comprise a filler. Preferably, the immediate release component a) and the prolonged-release component b) comprise a filler.
(15) If the pharmaceutical composition is according to (14), the filler can be at least one independently selected from the group consisting of anhydrous or monohydrate lactose, sucrose, microcrystalline cellulose, starch such as maize starch, pregelatinized starch, microcrystalline cellulose coated with colloidal silica, mannitol and any combination thereof, preferably the filler is at least one independently selected from the group consisting of anhydrous or monohydrate lactose, mannitol, microcrystalline cellulose, maize starch, saccharose and any combination thereof, and more preferably the filler is at least one selected from the group consisting of lactose monohydrate, microcrystalline cellulose, or a combination thereof.
(16) If the pharmaceutical composition is according to (14) or (15), the immediate release component a) can contain the filler in an amount of 10 to 50 wt.%, preferably 10 to 30 wt.%, based on the weight of the immediate release component a), and/or the modified-release component b) can contain the filler in an amount of 10 to 45 wt.%, preferably 10 to 30 wt.%, based on the weight of the modified-release component b).
(17) In the pharmaceutical composition, the immediate release component a) and/or the modified-release component b) can comprise a binder. Preferably, the immediate release component a) and the modified-release component b) comprise a binder.
(18) If the pharmaceutical composition is according to (17), the binder can be at least one independently selected from the group consisting of hydroxypropylcellulose, hydroxypropylmethylcellulose, methylcellulose, carboxymethylcellulose, hydroxycellulose, hydroxyethylcellulose, polyvinyl alcohol, polyvinylpyrrolidone, copovidone, pregelatinized starch, gelatine and any combination thereof, preferably the binder is at least one independently selected from the group consisting of hydroxypropyl cellulose, polyvinylpyrrolidone and any combination thereof, and more preferably the binder is polyvinylpyrrolidone.
(19) If the pharmaceutical composition is according to (17) or (18), the immediate release component a) can contain the binder in an amount of 0.4 to 5 wt.%, preferably 0.5 wt.%, based on the weight of the immediate release component a), and/or the modified-release component b) can contain the binder in an amount of 0.4 to 5 wt.%, preferably 0.9 wt.%, based on the weight of the modified-release component b).
(20) In the pharmaceutical composition, the immediate release component a) and/or the modified-release component b) may comprise a disintegrant. Preferably, the immediate release component a) and the modified-release component b) comprise a disintegrant.
(21) If the pharmaceutical composition is according to (20), the disintegrant can be at least one independently selected from the group consisting of carboxymethylcellulose sodium salt, sodium croscarmellose, microcrystalline cellulose, crospovidone, sodium starch glycolate, maize starch, and any combination thereof, preferably the disintegrant is at least one independently selected from the group consisting of sodium starch glycolate, sodium croscarmellose, maize starch and any combination thereof, more preferably the disintegrant is sodium croscarmellose or sodium starch glycolate.
(22) If the pharmaceutical composition is according to (20) or (21), the immediate release component a) can contain the disintegrant in an amount of 0.5 to 6 wt.%, preferably 2 to 6 wt.%, more preferably 5 wt.%, based on the weight of the immediate release component a), and/or the modified-release component b) can contain the disintegrant in an amount of 0.3 to 5 wt.%, preferably 2 to 5 wt.%, more preferably 3 wt.%, based on the weight of the modified-release component b).
(23) In the pharmaceutical composition, the immediate release component a) and/or the modified-release component b) can comprise a lubricant. Preferably, the immediate release component a) and the modified-release component b) comprise a lubricant.
(24) If the pharmaceutical composition is according to (23), the lubricant can be at least one independently selected from the group consisting of stearic acid, magnesium stearate, calcium stearate, sodium stearyl fumarate, glycerol tristearate, palm oil derivatives such as palmitic acid or hydrogenated palm oil, hydrogenated vegetable oil, such as hydrogenated castor oil, and any combination thereof, preferably the lubricant is at least one independently selected from magnesium stearate and stearic acid.
(25) If the pharmaceutical composition is according to (23) or (24), the immediate release component a) can contain the lubricant in an amount of 2 to 4 wt.%, preferably 2.3 wt.%, based on the weight of the immediate release component a), and/or the modified-release component b) can contain the lubricant in an amount of 0.5 to 2 wt.%, preferably 1 wt.%, based on the weight of the modified-release component b).
(26) In the pharmaceutical composition, the immediate release component a) and/or the modified-release component b) can comprise a glidant. Preferably, the immediate release component a) but not the modified-release component b) comprises a glidant.
(27) If the pharmaceutical composition is according to (26), the glidant can be at least one independently selected from the group consisting of fumed silica (colloidal silicon dioxide), such as colloidal anhydrous silica, starch, talc and any combination thereof, preferably the glidant is colloidal anhydrous silica.
(28) If the pharmaceutical composition is according to (26) or (27), the immediate release component a) can contain the glidant in an amount of 1.4 to 1.7 wt.%, preferably 1.6 wt.%, based on the weight of the immediate release component a), and/or the modified-release component b) can contain the glidant in an amount of up to 1.3 wt.%, based on the weight of the modified-release component b), and preferably the modified-release component b) contains no glidant.
(29) It is preferred that the immediate release component a) comprises 10 to 50 wt.%, preferably 10 to 30 wt.%, of a filler, 0.5 to 6 wt.%, preferably 2 to 6 wt.%, of a disintegrant, 1.4 to 1.7 wt.%, preferably 1.6 wt.%, of a glidant, 2 to 4 wt.%, preferably 2.3 wt.%, of a lubricant, based on the total weight of the immediate release component a).
(30) In the pharmaceutical composition, the immediate release component a) can comprise 0.4 to 5 wt.%, preferably 0.4 to 3 wt.%, more preferably 0.5 wt.%, of a binder, based on the total weight of the immediate release component a).
(31) It is preferred that the modified-release component b) comprises 10 to 40 wt.%, preferably 10 to 35 wt.%, more preferably 20 to 30 wt.%, of the controlled-release agent, 10 to 45 wt.%, preferably 10 to 30 wt.%, of a filler, 0.3 to 5 wt.%, preferably 2 to 5 wt.%, more preferably 3 wt.%, of a disintegrant, 0.4 to 5 wt.%, preferably 0.9 wt.%, of a binder, 0.5 to 2 wt.%, preferably 1 wt.%, of a lubricant, and, optionally, up to 1.3 wt.% of a glidant and preferably no glidant, based on the total weight of the modified-release component b).
(32) It is preferred that the immediate release component a) contains 45.5 wt.% of furazidin, 29.5 wt.% of microcrystalline cellulose, 5.0 wt.% of sodium starch glycolate, 15.6 wt.% of maize starch, 1.6 wt.% of colloidal anhydrous silica, 2.3 wt.% of stearic acid and 0.5 wt.% of polyvinylpyrrolidone, based on the total weight of the immediate release component a).
(33) It is preferred that the modified-release component b) contains 53.6 wt.% of furazidin, 13.6 wt.% of lactose monohydrate, 19.7 wt.% of hydroxypropylmethylcellulose K4M, 8.2 wt.% of hydroxyethylcellulose, 3 wt.% of croscarmellose sodium, 1.0 wt.% of magnesium stearate and 0.9 wt.% of polyvinylpyrrolidone, based on the total weight of the immediate release component b).
(34) After immersing the modified-release component b) in 30 to 35 mL of phosphate buffer of pH=4.5 for 2 hours, the buffer being contained in a beaker, heated by a wiggled, heated bath at 37°C, the matrix of the modified-release component b) is in a swollen state and can have a thickness in the range of 4.1 to 6.6 mm, preferably 4.9 to 5.5 mm.
(35) The pharmaceutical composition can be configured to provide, after oral administration, a maximum plasma concentration of furazidin (Cₘₐₓ) of 353.1±122.5 ng/mL after tₘₐₓ of 2.5±0.5 hours after administration, and an area under the plasma concentration-time curve from time zero to 24 hours (AUC₀₋₂₄) of 1404.1±392.8 ng·h/mL, as determined by human PK studies.
(36) The pharmaceutical composition can be configured to provide, after oral administration of a single dosage form of the pharmaceutical composition twice a day and 12 hours apart for 3 days, a maximum plasma concentration in steady state (C_{max,ss}) of furazidin of 516.4±149.3 ng/mL and an area under the plasma concentration-time curve from time zero to 24 hours in steady state (AUC_{0-24,ss}) of 3498.1±932.2 ng·h/mL, as determined by human PK studies.
(37) The pharmaceutical composition preferably has an in vitro dissolution rate of furazidin of 10 to 30 % within 1 h, based on the total furazidin content of the single dosage form, as determined by using European Pharmacopoeia edition 10 Apparatus Baskets "1" in 1000 mL volume of phosphate buffer solution of pH=6.8 with the addition of 2.5% CTAB (hexadecyltrimethylammonium bromide) at 37.0°C±0.5°C and 100 rpm.
(38) According to a further aspect, the present invention relates to the pharmaceutical composition according to one of the above embodiments for use in a method of treatment of a urinary tract infection.
(39) The urinary tract infection can be an acute or recurrent uncomplicated urinary tract infection, such as infections in women caused by *Escherichia coli.*
(40) The pharmaceutical composition can be administered orally two times daily, preferably 11 to 13 hours apart, more preferably 12 hours apart.
(41) The pharmaceutical composition can be administered orally within an interval of from 30 min before a meal and 30 min after a meal, preferably between at a meal and 30 min after a meal.
(42) The pharmaceutical composition can be administered for 5 to 8 days, preferably 7 days.
(43) Oral administration of one single dosage form of the pharmaceutical composition can provide a maximum plasma concentration of furazidin (Cₘₐₓ) of 353.1±122.5 ng/mL after tₘₐₓ of 2.5±0.5 hours after administration, and an area under the plasma concentration-time curve from time zero to 24 hours (AUC₀₋₂₄) of 1404.1±392.8 ng·h/mL, as determined by human PK studies.
(44) Oral administration of a single dosage form of the pharmaceutical composition twice a day and 12 hours apart can provide after 3 days a maximum plasma concentration in steady state (C_{max,ss}) of furazidin of 516.4±149.3 ng/mL and an area under the plasma concentration-time curve from time zero to 24 hours in steady state (AUC_{0-24,ss}) of 3498.1±932.2 ng·h/mL, as determined by human PK studies.

The prolonged-release pharmaceutical composition according to the present invention provides patients with an effective, safe and convenient therapy of UTI. Due to the prolonged release, the administration frequency of a single dosage form of the pharmaceutical composition can be reduced to two times a day (BID), for example, for 5 to 8 days such as 7 days. The pharmaceutical composition can be conveniently taken 11 to 13 hours, preferably 12 hours, apart and in combination with a meal, that is, shortly before, together with or shortly after a meal (in particular, breakfast and dinner). Here "shortly" means within 30 minutes. The meals can be consumed at regular times (for example, 7 a.m. and 7 p.m.), without significantly adjusting the eating times to the dosage regimen.

Patients using such a therapy would find it easier to take the drug regularly as scheduled, leading to an increase of the compliance. Furthermore, the prolonged release form of furazidin of the present invention provides more appropriate pharmacokinetic characteristics and, thus, improved efficacy compared to the immediate release tablets. The prolonged release form ensures maintaining a therapeutic level of the active substance over the time and, thus, enables twice a day (BID) administration. This is beneficial from both the clinical and the patient's perspective, since there is no need for continuous control of administration of subsequent drug doses. Constant therapeutic blood and urine concentrations of the furazidin are maintained during the treatment and, by consequence, increased treatment effectiveness, which may lead to decreased recurrence or complication rate, is observed. Additionally, the prolonged release dosage form prevents a rapid release of large quantities of furazidin and, thus, helps to minimise adverse effects such as the "first pass effect".

By deteriorating circumstances of UTI patients, the pharmaceutical composition of the present invention also increases the patients' quality of life. The present invention provides a prolonged-release pharmaceutical composition of furazidin, which provides better efficacy and safety profile than conventional immediate release furazidin tablets.

The efficacy and pharmacokinetics (PK) of the pharmaceutical composition according to the present invention have been confirmed in Phase I, pivotal, randomized, open-label, three-period, cross-over, food effect study under fasting and fed (high protein meal and high calorie/high fat meal) conditions and Phase I, pivotal, randomized, open-label, two-period, multiple dose, cross-over bioavailability study under fed conditions.

### Detailed description

According to an aspect of the present invention, a prolonged-release pharmaceutical composition for oral administration is provided, the prolonged-release pharmaceutical composition comprising: a) an immediate release component, comprising furazidin and one or more pharmaceutically acceptable excipients; and b) a modified-release component, comprising furazidin, a controlled-release agent, and one or more pharmaceutically acceptable excipients.

An immediate release (IR) component releases furazidin with no special rate controlling features, whereas the modified-release (MR) component releases furazidin with a delay after its administration due to the controlled-release agent. The understanding of the terms correspond to common pharmaceutical handbook definitions. The immediate release component shows a release of the active substance, which is not deliberately modified by a special formulation and/or manufacturing method. The immediate-release (IR) component ensures that furazidin is available to the body without relevant impact of the dosage form. According to the present invention, the IR component achieves in vitro dissolution of at least 80 wt.% of the furazidin within 45 minutes, based on the total furazidin content of the IR component. The modified-release component releases the active ingredient at a controlled rate, in particular, a constant rate. Preferably, the release rate is independent from the pH, the ionic content, and other contents within the entire segment of the gastrointestinal tract, in particular the stomach and the small intestine. According to the present invention, the modified-release component has an in vitro dissolution rate of 5 to 15 wt.% of furazidin within 2 h, based on the total furazidin content of the MR component. The 2-h time point of the dissolution profile has been chosen as representative for the correlation and referent for the prediction of the required full dissolution profile (up to release of the total amount of the API). The aforementioned in vitro dissolution rates are determined by using European Pharmacopoeia edition 10 Apparatus Baskets "1" in 1000 mL volume of phosphate buffer solution of pH=6.8 with the addition of 2.5% CTAB (hexadecyltrimethylammonium bromide) at 37.0°C±0.5°C and 100 rpm.

The combination of IR component and MR component according to the present invention provides a prolonged-release pharmaceutical composition. The prolonged-release dosage form shows a sustained release of furazidin compared to that of prior art immediate release dosage forms administered by the same route. The prolonged-release pharmaceutical composition achieves in vitro dissolution rates of furazidin of 10 to 40 wt.%, preferably 10 to 30 %, within 1 h, 40 to 65 wt.% within 6 h and above 80 wt.% within 14 h, based on the total furazidin content of a single dosage form of the pharmaceutical composition. The in vitro dissolution rates are determined by using European Pharmacopoeia edition 10 Apparatus Baskets "1" in 1000 mL volume of phosphate buffer solution of pH=6.8 with the addition of 2.5% CTAB (hexadecyltrimethylammonium bromide) at 37.0°C±0.5°C and 100 rpm.

The inventors have found that a prolonged, in particular more constant, release of furazidin can be achieved by the combination of IR component and MR component, as the immediate release is combined with a modified, controlled release. In other words, a component having a high release rate is combined with a component having a prolonged release rate as reflected by the in vitro dissolution rates of furazidin of the IR and MR components. More precisely, the prolonged-release pharmaceutical composition according to the invention may be understood as a biphasic release dosage form, in which the first phase of drug release is determined by a fast release dose fraction (immediate release component) providing a therapeutic drug level shortly after administration. The second extended release phase provides the dose fraction required to maintain an effective therapeutic level for a prolonged period. The pharmacokinetics of the pharmaceutical composition of the present invention thus differ fundamentally from the pharmacokinetics of prior art furazidin compositions for the treatment of UTI.

The inventors have surprisingly found that, due to the combination of IR component and MR component, having different furazidin release properties, the dosage frequency can be reduced to 2 times daily (BID), whereas prior art furazidin dosage forms require regular administration 3 or 4 times a day. Moreover, the pharmaceutical composition can be conveniently administered in combination with a meal (that is, shortly before, together with or shortly after the meal), for example breakfast and dinner, without significantly adjusting the times when the meal is taken or which food is consumed. Furazidin compositions of the prior art require adjusting the eating times to the dosage regimen (e.g. 8 hours apart) and the intake of protein-rich food to improve the furazidin availability. Thus, the pharmaceutical composition of the present invention enhances the patients' compliance as the pharmaceutical composition needs to be administered less often and the therapy is more convenient.

Since furazidin is released in a controlled, in particular, constant manner, therapeutic plasma and urine concentrations of furazidin can be achieved more easily, which improves the therapeutic effect. In particular, the furazidin concentration can be maintained above the MIC values. Since only a part of the furazidin is released immediately from the pharmaceutical composition and then small doses are released from the modified-release component, the release of large furazidin quantities is avoided. The release of small quantities of furazidin minimizes adverse effects, such as the "first pass effect".

In conclusion, the pharmaceutical composition of the present invention enables an effective, safe and convenient therapy of UTI. Compliance and efficacy are improved, and side-effects are reduced in comparison to furazidin compositions of the prior art.

In the pharmaceutical composition, furazidin may be contained as the free base or as a pharmaceutically acceptable salt thereof. Examples of pharmaceutically acceptable salts are the sodium or the potassium salts of furazidin. Since the salts have a higher solubility, it is preferred that the MR component contains furazidin as the free base. More preferably, the pharmaceutical composition contains furazidin as the free base and no salts of furazidin.

The pharmaceutical composition is a single dosage form in the form of a bilayer tablet. In the tablet, the immediate release component a) forms a first layer, the modified-release component b) forms a second layer, and the first layer at least partially covers the surface of the second layer. The tablet is a bilayer tablet, which means that the first layer is arranged on one side (i.e. one of the two faces) of the second layer and, typically, covers this side completely. Such a tablet can be produced economically.

The tablet may be uncoated or coated. The coating may, for example, be a film-coating. The tablet may have any suitable coating, e.g. functional coating. Such coatings can be found in the "Pharmaceutical Manufacturing Handbook" edited by Shayne Cox Gad, published by John Wiley & Sons, Inc., Hoboken, New Jersey, March 2008. Preferably, the tablet is uncoated.

In the pharmaceutical composition, the IR component a) and the MR component b) may be compressed together, for example, by using a bilayer-tableting machine. The IR component and the MR component may also be granulated and then be compressed to a tablet, for example, by using a bilayer-tableting machine. Preferably, the immediate release component a) is contained as granulates, and the modified-release component b) is contained as granulates, in the pharmaceutical composition. The granulates may be obtained by common dry or wet granulation techniques, preferably by wet granulation. In addition to granulates, the immediate release component a) and/or the modified-release component b) may also contain an extragranular fraction, for example, comprising one or more of a lubricant, a filler, an absorbent and a glidant. The components of an extragranular fraction are added after formation of the granulates.

According to an embodiment, the pharmaceutical composition comprises, in total, 150 to 250 mg of furazidin, preferably 180 to 220 mg of furazidin, and more preferably 200 mg of furazidin. Thus, a single dosage form has a higher furazidin content than a furazidin composition according to the prior art (100 mg or 50 mg). In the pharmaceutical composition, the immediate release component a) may contain 35 to 55 mg of furazidin, and the modified-release component b) may contain 145 to 165 mg of furazidin. Preferably, the immediate release component a) contains 45 to 55 mg of furazidin, and the modified-release component b) contains 145 to 155 mg of furazidin. More preferably, the immediate release component a) contains 50 mg of furazidin, and the modified-release component b) comprises 150 mg of furazidin. The pharmaceutical composition according to this embodiment is particularly suitable for a dosage regimen, where a single dosage form is administered two times daily, preferably, 11 to 13 hours, more preferably 12 hours apart. It allows for particularly constant, therapeutic plasma and blood concentrations of furazidin, which improves the efficacy of the treatment of UCI and reduces adverse side-effects.

In the aforementioned embodiment, the immediate release component a) has, in particular, a furazidin content of 32 to 50 wt.%, preferably 41 to 50 wt.%, more preferably 45 to 46 wt.%, such as 45.45 wt.%, based on the total weight of the immediate release component a). Furthermore, the modified release component b) has, in particular, a furazidin content of 40 to 60 wt.%, preferably 52 to 56 wt.%, more preferably 54 to 55 wt.%, such as 53.57 wt.%, based on the total weight of the modified-release component b).

In the pharmaceutical composition, the modified-release component b) contains a controlled-release agent in order to modify and sustain the release of furazidin from the MR component. Due to the controlled-release agent, the MR component has the above in vitro release values. The immediate release component a) has no modified or prolonged release of furazidin as noted above. In the pharmaceutical composition, the controlled-release agent may be used in an amount of 4 to 35 wt.%, preferably 9 to 30 wt.%, more preferably 15 to 25 wt.%, based on the total mass of the composition. The modified-release component b) may comprise 10 to 40 wt.% of the controlled-release agent, preferably 10 to 35 wt.% and more preferably 20 to 30 wt.% of the controlled-release agent, based on the total weight of the modified-release component b).

The controlled-release agent is contained in a matrix of the modified-release component b). The matrix of the modified-release component b) may be formed by the controlled-release agent or the controlled-release agent and a binder. The controlled-release agent is at least one pH-independent polymer. Hence, the matrix of the MR component is preferably formed by the pH-independent polymer or the pH-independent polymer and a binder. In the MR component, furazidin is preferably dispersed or suspended in the matrix, such that the release is modified and prolonged.

The term "pH-independent polymer" means that the polymer matrix exhibits pH-independent drug permeability and pH-independent swelling and, thus, enables modified release of the active ingredient by pH-independent swelling and erosion. Thus, the release of furazidin from such a polymer matrix is independent from the pH environment and the modified release according to the above in vitro dissolution values is fulfilled irrespective of the pH. "pH-independent" means independent from the pH value in the gastrointestinal tract, which is usually between 1.5 and 8.5 (stomach and small intestine). More preferably, a thickness ratio of the thickness of the matrix of the pH-independent polymer in a swollen state to the thickness of the dry matrix of the pH-independent polymer before swelling is in the range of 1.1 to 1.8, preferably 1.3 to 1.6, wherein the swollen state is obtained by immersing the dry modified-release component b) in 30 to 35 mL of phosphate buffer of pH=4.5 contained in beaker for 2 h, the beaker being placed in a wiggled, heated bath at 37°C.

The pH-independent polymer is at least one selected from the group consisting of hydroxypropylmethylcellulose (HPMC), hydroxyethylcellulose (HEC), hydroxypropylcellulose, xanthan gum, sodium carboxymethylcellulose, non-ionic poly(ethylene oxide), and any combination thereof. More preferably, the pH-independent polymer has a weight average molecular weight (Mw) of 50,000 to 7,000,000, preferably 350,000 to 800,000. Preferred examples of hydroxypropylmethylcellulose (HPMC) have a weight average molecular weight of 70,000 to 1,000,000, preferably 70,000 to 600,000, more preferably 70,000 to 170,000. Preferred examples of carboxymethylcellulose (CMC) have a weight average molecular weight of 200,000 to 500,000, preferably 350,000 to 450,000, more preferably 370,000 to 400,000. Preferred examples of hydroxyethylcellulose (HEC) have a weight average molecular weight of 50,000 to 200,000, preferably 80,000 to 150,000, more preferably 80,000 to 100,000. The weight average molecular weights are measured by GPC/SEC according to EU Pharmacopoeia 5.0 method 2.2.30. In the modified-release component b), it is even more preferred if the pH-independent polymer is a combination of hydroxypropylmethylcellulose K4M and hydroxyethylcellulose such as Natrosol, a combination of hydroxypropylmethylcellulose K4M and non-ionic poly(ethylene oxide) such as Polyox 7 000 000, a combination of hydroxypropylmethylcellulose K4M and hydroxypropylmethylcellulose K100LV (Mw = 160,000 to 170,000, such as 164,000), a combination of hydroxypropylmethylcellulose K4M and hydroxypropylcellulose such as Klucel LF, a combination of hydroxypropylmethylcellulose K15M and xanthan gum such as Xantural 75, or a combination of hydroxypropylmethylcellulose K15M and sodium carboxymethylcellulose such as Blanose 7M31F. Combinations of two pH-independent polymers are preferred for more adequately sustaining and controlling the furazidin release. Most preferably, a combination of hydroxypropylmethylcellulose K4M and hydroxyethylcellulose is used as the pH-independent polymer.

In the pharmaceutical composition, the immediate release component a) and the modified-release component b) each comprise at least one excipient. As the at least one excipient, the immediate release component a) may comprise at least one of a filler, a disintegrant, a binder, a lubricant, a glidant and any combination thereof. Preferably, the immediate release component a) comprises a filler, a disintegrant, a glidant, a lubricant, and, optionally, a binder. The immediate release component a) preferably consists of furazidin and a filler, a disintegrant, a glidant, a lubricant, and, optionally, a binder. Preferably, a binder is also present in the immediate-release component a).

The modified-release component b) may comprise, as the at least one excipient, at least one of a filler, a disintegrant, a binder, a lubricant, a glidant and any combination thereof, preferably the modified-release component b) comprises a filler, a disintegrant, a binder, a lubricant, and, optionally, a glidant. The modified-release component b) preferably consists of furazidin, a controlled-release agent, a filler, a disintegrant, a binder, a lubricant, and, optionally, a glidant. More preferably, the MR component does not contain a glidant.

In the pharmaceutical composition, the immediate release component a) and/or, preferably and, the prolonged-release component b) comprise a filler. Herein, a filler means at least one filler. Accordingly, the filler may be at least one filler independently selected from the group consisting of anhydrous or monohydrate lactose, sucrose, microcrystalline cellulose, starch such as maize starch, pregelatinized starch, microcrystalline cellulose coated with colloidal silica, mannitol and any combination thereof. Preferably, the filler is at least one independently selected from the group consisting of anhydrous or monohydrate lactose, mannitol, microcrystalline cellulose, maize starch, saccharose and any combination thereof. More preferably, the filler is at least one selected from the group consisting of lactose monohydrate, microcrystalline cellulose, or a combination thereof. The immediate release component a) may contain the filler in an amount of 10 to 50 wt.%, preferably 10 to 30 wt.%, based on the weight of the immediate release component a). The modified-release component b) contains the filler in an amount of 10 to 45 wt.%, preferably 10 to 30 wt.%, based on the weight of the modified-release component b).

In the pharmaceutical composition, the immediate release component a) and/or the modified-release component b) comprise a binder. Preferably the immediate release component a) and the modified-release component b) comprise a binder, the binder being contained in addition to the controlled-release agent. Herein, a binder means at least one binder. Accordingly, the binder may, for example, be at least one binder independently selected from the group consisting of hydroxypropylcellulose, hydroxypropylmethylcellulose, methylcellulose, carboxymethylcellulose, hydroxycellulose, hydroxyethylcellulose, polyvinyl alcohol, polyvinylpyrrolidone, copovidone, pregelatinized starch, gelatine and any combination thereof. Preferably, the binder is at least one independently selected from the group consisting of hydroxypropyl cellulose, polyvinylpyrrolidone and any combination thereof. More preferably the binder is polyvinylpyrrolidone. The binder may be used in an amount equal to 0.4 to 5 wt.%, preferably 0.8 to 3 wt.%, more preferably 0.8 wt.%, based on the total mass of the composition. The immediate release component a) may contain the binder in an amount of 0.4 to 5 wt.%, preferably 0.4 to 3 wt.%, more preferably 0.5 wt.%, based on the weight of the immediate release component a). The modified-release component b) may contain the binder in an amount of 0.4 to 5 wt.%, preferably 0.4 to 3 wt.%, more preferably 0.9 wt.%, based on the weight of the modified-release component b). A binder is not required if the respective component is produced by dry granulation or direct compacting. It is however preferred to use wet granulation in the preparation of the components, where a binder is commonly used as described in more detail below.

In the pharmaceutical composition, the immediate release component a) and/or, preferably and, the modified-release component b) comprise a disintegrant. Herein, a disintegrant means at least one disintegrant. Accordingly, the disintegrant may, for example, be at least one disintegrant independently selected from the group consisting of carboxymethylcellulose sodium salt, sodium croscarmellose, microcrystalline cellulose, crospovidone, sodium starch glycolate, maize starch, and any combination thereof. Preferably, the disintegrant is at least one independently selected from the group consisting of sodium starch glycolate, sodium croscarmellose, maize starch and any combination thereof. More preferably, the disintegrant is sodium croscarmellose or sodium starch glycolate. The disintegrant may be used in an amount of 0.3 to 5 wt.%, preferably 1 to 4.5 wt.%, more preferably 3.6 wt.%, based on the total mass of the composition. The immediate release component a) may contain the disintegrant in an amount of 0.5 to 6 wt.%, preferably 2 to 6 wt.%, more preferably 5 wt.%, based on the weight of the immediate release component a). The modified-release component b) may contain the disintegrant in an amount of 0.3 to 5 wt.%, preferably 2 to 5 wt.%, more preferably 3 wt.%, based on the weight of the modified-release component b).

In the pharmaceutical composition, the immediate release component a) and/or, preferably and, the modified-release component b) comprise a lubricant. Herein, a lubricant means at least one lubricant. Accordingly, the lubricant may, for example, be at least one lubricant independently selected from the group consisting of stearic acid, magnesium stearate, calcium stearate, sodium stearyl fumarate, glycerol tristearate, palm oil derivatives such as palmitic acid or hydrogenated palm oil, especially Softisan 154, hydrogenated vegetable oil, such as hydrogenated castor oil, and any combination thereof. Preferably, the lubricant is at least one independently selected from magnesium stearate and stearic acid. The lubricant may be used in an amount of 1 to 10 wt.%, preferably 2 to 4 wt.%, more preferably 1.3 to 1.4 wt.% such as 1.35 wt.%, based on the total mass of the composition. The immediate release component a) may contain the lubricant in an amount of 2 to 4 wt.%, preferably 2.3 wt.%, based on the weight of the immediate release component a). The modified-release component b) may contain the lubricant in an amount of 0.5 to 2 wt.%, preferably 1 wt.%, based on the weight of the modified-release component b).

In the pharmaceutical composition, the immediate release component a) and/or the modified-release component b) may comprise a glidant. It is preferred that the immediate release component a) but not the modified-release component b) comprises a glidant. Herein, a glidant means at least one glidant. Accordingly, the glidant may, for example, be at least one glidant independently selected from the group consisting of fumed silica (colloidal silicon dioxide), such as colloidal anhydrous silica, starch, talc and any combination thereof. Preferably, the glidant is colloidal anhydrous silica. The glidant may be used in an amount of 0.3 to 3 wt.%, preferably 0.4 to 2 wt.%, more preferably 0.46 wt.%, based on the total mass of the composition. The immediate release component a) may contain the glidant in an amount of 1.4 to 1.7 wt.%, preferably 1.6 wt.%, based on the weight of the immediate release component a). The modified-release component b) may contain the glidant in an amount of up to 1.3 wt.%, based on the weight of the modified-release component b).

According to a further embodiment of the pharmaceutical composition, the immediate release component a) and/or the modified-release component b) comprise at least one absorbent as an excipient. An absorbent may, for example, be added to the granulate of the IR component or the MR component or the extragranular fraction in order to stabilize moisture level and, thus, to improve the quality of the physical properties of the granulate and facilitate subsequent compression of the granulate to form a tablet. The absorbent provides stabilisation of moisture level and pH of the granulate and of the final composition. The absorbent is preferably pH-neutral. The absorbent may, for example, be selected from magnesium aluminometasilicates. Preferably, the absorbent is selected from one of the Neusilin grades, such as Neusilin US2^{®} from Fuji Chemicals. In general, an absorbent is not required in the pharmaceutical composition.

Particularly preferred pharmaceutical compositions of the present invention are obtained by combining preferred embodiments such as preferred contents and preferred components.

For example, according to a particularly preferred embodiment, the single dosage form of the pharmaceutical composition contains 150 to 250 mg of furazidin, preferably 180 to 220 mg of furazidin, and more preferably 200 mg of furazidin. The immediate release component a) comprises 35 to 55 mg, preferably 45 to 55 mg, more preferably 50 mg, of furazidin, and the modified-release component b) comprises 145 to 165 mg, preferably 145 to 155 mg, more preferably 150 mg, of furazidin. In the matrix of the modified-release component b), the modified-release component b) comprises 10 to 40 wt.%, preferably 10 to 35 wt.%, more preferably 20 to 30 wt.%, of the pH-independent polymer as the controlled-release agent.

According to another particularly preferred embodiment, the immediate release component a) comprises 10 to 50 wt.%, preferably 10 to 30 wt.%, of a filler, 0.5 to 6 wt.%, preferably 2 to 6 wt.%, of a disintegrant, 1.4 to 1.7 wt.%, preferably 1.6 wt.%, of a glidant, 2 to 4 wt.%, preferably 2.3 wt.%, of a lubricant and, optionally, 0.4 to 5 wt.% of a binder, based on the total weight of the immediate release component a). The immediate release component a) may comprise 0.4 to 5 wt.%, preferably 0.4 to 3 wt.%, more preferably 0.5 wt.%, of a binder, based on the total weight of the immediate release component a). The filler, the disintegrant, the lubricant, the glidant and the binder are preferably selected from the above-mentioned compounds. The immediate release component a) may further contain 32 to 50 wt.%, preferably 41 to 50 wt.%, more preferably 45 to 46 wt.%, such as 45.45 wt.%, of furazidin and can consist of furazidin and the aforementioned excipients in the indicated amounts. Even more preferably, the immediate release component a) contains or consist of 45.5 wt.% of furazidin, 29.5 wt.% of microcrystalline cellulose, 5.0 wt.% of sodium starch glycolate, 15.6 wt.% of maize starch, 1.6 wt.% of colloidal anhydrous silica, 2.3 wt.% of stearic acid and 0.5 wt.% of polyvinylpyrrolidone, based on the total weight of the immediate release component a).

According to another particularly preferred embodiment, the modified-release component b) comprises 10 to 40 wt.%, preferably 10 to 35 wt.%, more preferably 20 to 30 wt.%, of the controlled-release agent, 10 to 45 wt.%, preferably 10 to 30 wt.%, of a filler, 0.3 to 5 wt.%, preferably 2 to 5 wt.%, more preferably 3 wt.%, of a disintegrant, 0.4 to 5 wt.%, preferably 0.9 wt.%, of a binder, 0.5 to 2 wt.%, preferably 1 wt.%, of a lubricant, and, optionally, up to 1.3 wt.% of a glidant, based on the total weight of the modified-release component b). Preferably, it does not contain a glidant. The controlled-release agent, the filler, the disintegrant, the lubricant, the glidant and the binder are preferably selected from the above-mentioned compounds. The modified release component b) may further contain 40 to 60 wt.%, preferably 52 to 56 wt.%, more preferably 54 to 55 wt.%, such as 53.57 wt.%, of furazidin and can consist of furazidin and the consist of furazidin and the aforementioned excipients in the indicated amounts. Even more preferably, the modified-release component b) contains or consists of 53.6 wt.% of furazidin, 13.6 wt.% of lactose monohydrate, 19.7 wt.% of hydroxypropylmethylcellulose K4M, 8.2 wt.% of hydroxyethylcellulose, 3 wt.% of croscarmellose sodium, 1.0 wt.% of magnesium stearate and 0.9 wt.% of polyvinylpyrrolidone, based on the total weight of the immediate release component b).

In a preferred variant of the pharmaceutical composition, the modified release of furazidin may be further characterized by the thickness of the pH-independent-polymer matrix after swelling, i.e. in the swollen state. After immersing the modified-release component b) in 30 to 35 mL of phosphate buffer of pH=4.5 for 2 hours, the buffer being contained in a beaker, heated by a wiggled, heated bath at 37°C, the matrix of the modified-release component b) is in a swollen state and has a thickness in the range of 4.1 to 6.6 mm, preferably 4.9 to 5.5 mm. The thickness measurements are performed on the MR component, swollen in a medium resembling fed conditions in vivo. Such a thickness of the swollen MR component is favourable for obtaining a controlled release of furazidin from the pH-independent polymer matrix and absorption in the upper part of the small intestine in order to obtain a desirable time/concentration profile of furazidin. Pharmacokinetics studies (PK phase I studies) showed that furazidin in form of a prolonged release composition according to the present invention is mainly absorbed in the upper part of the small intestine. Before swelling, the dry modified-release component has a thickness in the range of 3.0 to 5.0 mm, preferably 3.5 - 4.0 mm. The thickness ratio of the thickness of the modified-release component b) in the swollen state to the thickness of the dry modified-release component b) before swelling is in the range of 1.1 to 1.8, preferably 1.3 to 1.6.

As outlined above, the pharmaceutical composition of the present invention provides preferable pharmacokinetics as a controlled, preferably constant, release of furazidin is achieved. The pharmacokinetics obtained with the pharmaceutical composition of the present invention differ fundamentally from prior art furazidin compositions for the treatment of UTI, which basically show an immediate release of furazidin. The preferable pharmacokinetics allow for reducing the dosage frequency in the treatment of UTI, thus improving patients' compliance and convenience and ensuring a better therapeutic efficacy.

According to a preferred embodiment, the pharmaceutical composition is configured to provide, after oral administration, a maximum plasma concentration of furazidin (Cₘₐₓ) of 353.1±122.5 ng/mL after tₘₐₓ of 2.5±0.5 hours after administration, and an area under the plasma concentration-time curve from time zero to 24 hours (AUC₀₋₂₄) of 1404.1±392.8 ng·h/mL, as determined by human PK studies. Furthermore, the pharmaceutical composition may be configured to provide, after oral administration of a single dosage form of the pharmaceutical composition twice a day and 12 hours apart for 3 days, a maximum plasma concentration in steady state (C_{max,ss}) of furazidin of 516.4±149.3 ng/mL and an area under the plasma concentration-time curve from time zero to 24 hours in steady state (AUC_{0-24,ss}) of 3498.1±932.2 ng·h/mL, as determined by human PK studies.

The pharmaceutical composition of the present invention has an in vitro dissolution rate of furazidin of 10 to 40 wt.%, preferably 10 to 30 %, within 1 h, 40 to 65 wt.% within 6 h and above 80 wt.% within 14 h, based on the total furazidin content of the single dosage form, as determined by using European Pharmacopoeia edition 10 Apparatus Baskets "1" in 1000 mL volume of phosphate buffer solution of pH=6.8 with the addition of 2.5% CTAB (hexadecyltrimethylammonium bromide) at 37.0°C±0.5°C and 100 rpm.

A method of manufacturing the prolonged-release pharmaceutical composition, which is not encompassed by the wording of the claims, enables the production of the pharmaceutical composition using conventional tools and well-known pharmaceutical technologies, while significantly simplifying the manufacturing process, and reducing its costs comparing to manufacturing standards known in the art. The pharmaceutical composition in the form of a bilayer tablet can be prepared by a method comprising direct compression, dry granulation or wet granulation for making the immediate-release component and the modified-release component b).

Thus, a method of manufacturing the prolonged-release pharmaceutical composition comprises (a) a step of preparing the immediate release component a); (b) a step of preparing the modified-release component b); (c) a step of combining the immediate release component a) and the modified-release component (b) to form a bilayer tablet, in which the immediate release component a) forms a first layer, the modified-release component (b) forms a second layer; and, optionally, (d) a step of forming a coating around the first and second layers obtained in step (c). Preferably, no coating is formed, that is, step (d) is preferably not carried out.

In step (a) and step (b), the IR component and MR component may be independently prepared using a dry or wet technology such as dry or wet granulation.

A dry-granulated IR component or MR component can be obtained using direct dry granulation of a powder mixture comprising furazidin or a pharmaceutically acceptable salt thereof, suitable excipients as described above and, in case of the MR component, a controlled-release agent, as described above, in particular at least one pH-independent polymer. Other excipients can be also included, if needed. Suitable excipients, used in pharmacy, can be found in the "Handbook of Pharmaceutical Excipients" edited by R. C. Rowe, P. J. Sheskey, and S. Owen, Pharmaceutical Press, edition VII. The dry granulation method used to obtain the IR component (step (a)) or the MR component (step (b)) can be selected from roller compaction and slugging. Methods known to the skilled person and are described, for example, in the "Pharmaceutical Manufacturing Handbook" edited by Shayne Cox Gad, published by John Wiley & Sons, Inc., Hoboken, New Jersey.

A wet-granulated IR component or MR component can be obtained using a suitable granulation method known in the art using powder mixtures comprising furazidin or a pharmaceutically acceptable salt thereof, suitable excipients as described above and, in case of the MR component, a controlled-release agent, as described above, in particular at least one pH-independent polymer. Other excipients can be also included, if needed. The wet granulation method used to obtain the IR component (step (a)) or the MR component (step (b)) can be selected from low shear, high shear, and fluid bed granulation. Preferably, the IR component and the MR component are obtained by wet granulation (steps (a) and (b)).

Blending operations can be performed in a suitable blender, preferably in a container blender.

According to an embodiment of the method, the IR component a) and/or the MR component b) are prepared using dry granulation (steps (a) and (b)). In such a variant, the method preferably comprises the following steps: (I) blending furazidin or a pharmaceutically acceptable salt thereof with all excipients except lubricant and, in case of the MR component, with the at least one controlled-release agent to homogeneity; II) adding a part of the lubricant and subsequent blending; III) dry granulation by roller-compaction or slugging; IV) breaking-down the resulting slugs or sheets using a milling technique to produce granules; V) adding the remaining part of lubricant and subsequent blending.

In the dry methods of granulation, the primary powder blend particles are aggregated under high pressure using one of two main dry granulation processes: either roller-compaction or slugging. In both cases the intermediate products, slugs or sheets, are broken down using a suitable milling technique to produce granular material, which can be additionally sieved to separate the desired size fraction. Such a way of processing a furazidin powder blend is very advantageous for several reasons. It allows for the elimination of water, which may affect furazidin stability, and obtaining a granulate having grain sizes that are optimal for further processing (combination with the second component comprising furazidin and optional extragranular phase component without the effect of physical segregation). Additionally, a broad ratio (wt.%) of furazidin can be blended into the dry granulate intermediate, which is not possible in other dry pharmaceutical processes (e.g. direct compression).

According to another embodiment of the method, the IR component a) and/or the MR component b) are prepared using wet granulation (steps (a) and (b)). In such a variant, the method preferably comprises the following steps: I) mixing of furazidin or a pharmaceutically acceptable salt thereof with at least one filler, at least one disintegrant, optionally further excipients, and, in case of the MR component, at least one controlled-release agent in a high-shear granulator to homogeneity; II) granulating the mixture of step I) by addition of a binder solution in water or another processing agent, such as an alcohol, e.g. ethanol, or with a mixture thereof; III) drying and unifying the dried granule sizes by sieving or screening; and, optionally, IV) adding an extragranular phase comprising lubricant and/or a mixture of filler and glidant and/or an absorbent and a glidant.

Furazidin may be also subjected to a process of wet granulation (e.g. low shear, high shear or in a fluidized bed), to improve its solubility. Wet granulation facilitates obtaining the desired in vitro and in vivo release profiles. The resulting wet granulate is dried and, for example, screened to obtain a proper distribution of the particles, allowing a durable combination with the other component and additional excipients.

According to a preferred embodiment of the method, the IR component a) is prepared using dry granulation (step (a)), and the MR component b) is prepared using wet granulation (step (b)). It is also preferred to prepare the IR component (step (a) and the MR component (step (b)) by wet granulation.

In step (c) of the method, the resulting components a) and b) are combined together, and depending on the desired final form, for example, compressed into separate layers in a tablet, optionally followed by forming a coating in step (d), resulting in a single unit dosage form. Preferably, the prolonged-release pharmaceutical composition is prepared by using a bilayer tableting technology in step (c).

According to another aspect, the present invention provides a pharmaceutical composition as described herein above for use in a method of treatment of a urinary tract infection. The urinary tract infection is preferably an acute or recurrent uncomplicated urinary tract infection, for example caused by *Escherichia coli,* such as recurrent uncomplicated urinary tract infection in women caused by *Escherichia coli.*

According to an embodiment, the pharmaceutical composition is administered orally two times daily, preferably 11 to 13 hours apart, more preferably 12 hours apart. Typically, the pharmaceutical composition is administered for 5 to 8 days, preferably 7 days. This regimen has the advantage of better patients' compliance and improved quality of life during therapy in comparison to the treatment of UCI using a prior art furazidin composition. In conventional therapies, the high dosages of furazidin require large dosage forms, with the necessity of taking 2 or 4 tablets, depending on the available presentation, at once 3 or 4 times daily for 7 up to 10 days.

Typically, the pharmaceutical composition is administered in combination with a meal, as absorption of furazidin is improved. In combination with a meal means that the pharmaceutical composition is taken shortly before, together with or after the meal. It is thus preferred that the pharmaceutical composition is a single dosage form, which is administered orally within an interval of from 30 min before a meal and 30 min after a meal. Preferably, the pharmaceutical composition is administered between at a meal and 30 min after a meal, and more preferably, right after the meal, that is, within 5 min after a meal.

For oral administration two times a day, a single dosage form of the pharmaceutical composition usually contains 150 to 250 mg of furazidin, preferably 180 to 220 mg of furazidin, and more preferably 200 mg of furazidin. In particular, the immediate release component a) comprises 35 to 55 mg, preferably 45 to 55 mg, more preferably 50 mg, of furazidin, and the modified-release component b) comprises 145 to 165 mg, preferably 145 to 155 mg, more preferably 150 mg, of furazidin. In a particularly preferred variant, 200 mg of furazidin formulated as a fixed dose comprising 50 mg of furazidin in IR component and 150 mg of furazidin in MR component is administered, 2 times daily, for example, for 7 days.

It is an advantage of the present invention that the prolonged-release furazidin composition provides continuous furazidin effective concentration levels during the whole period of treatment, especially during the night. This effect could not be achieved with the use of a prior art immediate release formulation, which provides effective concentrations only during a short period of time after administration. Consequently, continuous bacteriostatic concentration in serum and urine could not be provided, which is however crucial for effective therapy.

The composition for use in the method of treatment according to the present invention thus provides favourable furazidin pharmacokinetics, which are not obtained by prior art furazidin compositions. Preferably, wherein oral administration of one single dosage form of the pharmaceutical composition provides a maximum plasma concentration of furazidin (Cₘₐₓ) of 353.1±122.5 ng/mL after tₘₐₓ of 2.5±0.5 hours after administration, and an area under the plasma concentration-time curve from time zero to 24 hours (AUC₀₋₂₄) of 1404.1±392.8 ng·h/mL, as determined by human PK studies described below. It is further preferred that oral administration of a single dosage form of the pharmaceutical composition twice a day and 12 hours apart provides after 3 days a maximum plasma concentration in steady state (C_{max,ss}) of furazidin of 516.4±149.3 ng/mL and an area under the plasma concentration-time curve from time zero to 24 hours in steady state (AUC_{0-24,ss}) of 3498.1±932.2 ng·h/mL, as determined by human PK studies as described below.

### Brief description of the drawings

Fig. 1 shows the pharmacokinetic profiles of means in plasma of the food effect study of Example 5.
Fig. 2 shows the pharmacokinetic profiles of means in urine of the food effect study of Example 5.
Fig. 3 shows the pharmacokinetic profile of means in plasma of the multiple-dose study of Example 6.
Fig. 4 shows the pharmacokinetic profile of means in urine of the multiple-dose study of Example 6.

### Examples

### Example 1. Preparation of IR component, MR component and pharmaceutical composition

Exemplary IR components (IR/1 to IR/4) and MR components (MR/1 to MR/9) according to the present invention are presented in the Tables 1 and 2 below.

**Table 1. Compositions of exemplary IR components**

| | wet granulation | | | | direct tabletting | | | |
|---|---|---|---|---|---|---|---|---|
| | **Example IR/1** | | **Example IR/2** | | **Example IR/3** | | **Example IR/4** | |
| IR Component | mg | wt.% | mg | wt.% | mg | wt.% | mg | wt.% |
| Furazidin | 50.00 | 45.45 | 50.00 | 45.45 | 50.00 | 41.67 | 50.00 | 50.00 |
| Microcrystalline cellulose, type 102 | 32.44 | 29.49 | 32.45 | 29.50 | 38.00 | 31.67 | | |
| Saccharose | | | | | | | 13.75 | 13.75 |
| Sodium starch glycolate, Ultraamylopectin | 5.50 | 5.0 | | | 4.0 | 3.33 | | |
| Croscarmellose Sodium | | | 5.50 | 5.00 | | | | |
| Polyvinylpyrrolidone K90 | 0.56 | 0.51 | | | | | | |
| Polyvinylpyrrolidone K30 | | | 3.85 | 3.5 | | | | |
| Maize starch | 17.20 | 15.64 | 13.90 | 12.64 | 21.20 | 17.66 | 27.50 | 27.50 |
| Silica, colloidal anhydrous | 1.80 | 1.64 | 1.80 | 1.64 | 1.80 | 1.50 | 1.25 | 1.25 |
| Stearic acid | 2.50 | 2.27 | | | 5.00 | 4.17 | 7.5 | 7.5 |
| Sodium stearyl fumarate | | | 2.50 | 2.27 | | | | |
| immediate release component weight | 110.00 | 100.00 | 110.00 | 100.00 | 120.00 | 100.00 | 100.00 | 100.00 |

**Table 2. Compositions of exemplary MR components**

| | mg/MR component | wt.%/MR component | | | | | | |
|---|---|---|---|---|---|---|---|---|
| | **Example MR/1** | **Example MR/1** | **Example MR/2** | **Example MR/3** | **Example MR/4** | **Example MR/5** | **Example MR/6** | **Example MR/7** |
| **Furazidin** | 150.00 | 53.58 | 53.58 | 53.58 | 53.58 | 53.58 | 53.58 | 53.56 |
| Lactose, monohydrate 200 | 38.00 | 13.57 | 13.57 | 13.57 | 13.57 | 13.57 | 13.57 | 15.57 |
| Methocel K4M Hydroxypropylmethylocellulose K4M Hypromellose, type K4M | 55.1 | 19.68 | | 19.68 | | | | 19.68 |
| Methocel K15M Hydroxypropylmethylcellulose K15M Hypromellose, type K15M | | | | | | 23.6 | 23.6 | |
| Hydroxyethylcellulose, type 250L Natrosol 250 L | 23 | 8.21 | | | | | | 5.21 |
| Xanthan gum Xantural 75 | | | 27.86 | | | 4.29 | | |
| Hydroxypropylcellulose Klucel LF | | | | 8.21 | | | | |
| Sodium carboxymethylcellulose Blanoza 7M31F | | | | | 27.86 | | 4.29 | |
| Polyvinylpyrrolidone K90 | 2.55 | 0.91 | 0.91 | 0.91 | 0.91 | 0.91 | 0.91 | 0.91 |
| Croscarmellose Sodium | 8.55 | 3.05 | 3.05 | 3.05 | 3.05 | 3.05 | 3.05 | 3.05 |
| Magnesium stearate | 2.80 | 1.0 | 1.0 | 1.0 | 1.0 | 1.0 | 1.0 | 1.0 |
| Modified-release component weight | 280.0 | 100 | 100 | 100 | 100 | 100 | 100 | 100 |

**Table 2 (continued). Composition of exemplary MR components**

| | **Example MR/8** | | **Example MR/9** | |
|---|---|---|---|---|
| | mg/MR component | wt.%/MR component | mg/MR component | wt.%/MR component |
| Furazidin | 150.00 | 54.55 | 130.00 | 47.27 |
| Lactose, Monohydrate200 | 27.53 | 10.00 | 47.53 | 17.28 |
| Methocel K4M Hypromellose, type K4M | 27.0 | 9.82 | 27.00 | 9.82 |
| Povidone K30 Polyvinylpyrrolidone K30 | 10.12 | 3.68 | 10.12 | 3.68 |
| Neusilin S2 | 27.5 | 10.0 | 27.50 | 10.00 |
| Croscarmellose Sodium | 9.6 | 3.49 | 9.60 | 3.49 |
| Polyox, WSR303 LEO NF | 20.5 | 7.46 | 20.50 | 7.46 |
| Magnesium stearate | 2.75 | 1.0 | 2.75 | 1.00 |
| modified release component weight | 275.0 | 100 | 275.0 | 100.0 |

In the following procedures, the amounts of the components were used as specified in Tables 1 and 2.

### Preparation of granulate of immediate release component

The IR components IR/1 and IR/2 are prepared as follows:
1. Weighing of furazidin, microcrystalline cellulose 102, sodium starch glycolate, povidone 90, maize starch, colloidal anhydrous silica, stearic acid (i.e. furazidin, filler, disintegrant, binder, glidant, lubricant).
2. Mixing furazidin, microcrystalline cellulose 102, sodium starch glycolate, a part of the maize starch (72 wt.%), in a high shear granulator (i.e. furazidin, filler, disintegrant).
3. Preparation of the binder solution: Povidone 90, purified water (i.e. binder, processing agent)
4. Wet granulation using the mixture from step 2 and the binder solution from step 3 is performed in a High Shear Granulator. Granulation time 1 to 3 min. Appearance of the wet blend was assessed by squeezing the mass.
5. Wet-sizing of the granulate obtained in step 4 in a sizing machine, IPC - Screen size 3 to 6 mm.
6. Drying of the seized granulate obtained step 5 in a fluid bed dryer at a temperature of max. 55°C, accepted levels of LOD are in a range of 1 to 3%.
7. Sizing of the granulate obtained in step 6 in a sizing machine , IPC - Screen size 1 mm
8. Weighing of the remaining part of the maize starch (28 wt.%), colloidal anhydrous silica, stearic acid (i.e. filler, lubricant, glidant).
9. Blending the granulate obtained in step 7 with the part of maize starch, colloidal anhydrous silica (i.e. filler, glidant) after sizing.
10. Blending the sized granulate from step 9 with stearic acid (lubricant) in a bin blender. Blending time 3 to 5 min.

The IR components IR/3 and IR/4 are prepared as follows:
1. Weighing of furazidin, microcrystalline cellulose 102 or saccharose, maize starch, colloidal anhydrous silica, stearic acid (i.e. furazidin, filler, disintegrant, glidant, lubricant).
2. Blending all the components from step 1 except of the lubricant.
3. Blending the blend obtained in step 2 with the lubricant.
4. Tabletting on a tablet press.

### Preparation of granulate of modified release component

The MR components MR/1 to MR/9 are prepared as follows:
1. Weighing of furazidin, lactose monohydrate, hypromellose K4M, povidone 90, hydroxyethylcellulose 250L, croscarmellose sodium, magnesium stearate (i.e. furazidin, filler, control release agent, binder, lubricant and disintegrant).
2. Mixing furazidin, lactose monohydrate, hypromellose K4M, hydroxyethylcellulose 250L, croscarmellose sodium in a high shear granulator (i.e. furazidin, filler, control release agent and disintegrant).
3. Preparation of the binder solution: Povidone 90, purified water, ethanol (i.e. binder and processing agents)
4. Wet Granulation using the mixture from step 2 and the binder solution from step 3 is performed in a high shear granulator. Granulation time 1 to 3 min. Appearance of the wet blend was assessed by squeezing the mass.
5. Wet-sizing of the granulate obtained in step 4 in a sizing machine, IPC - Screen size 3 to 6 mm.
6. Drying of the seized granulate obtained in step 5 in a fluid bed dryer at a temperature of max. 55°C, accepted levels of LOD are in a range of 1 to 3%.
7. Blending of the sized granulate from step 6 with magnesium stearate (lubricant) in a bin blender. Blending time 3 to 5 min.

### Preparation of bilayer tablets

The final blend granulates are compressed into bilayer-tablets with the aid of rotary press machine. During the compression process, the following requirements of the tablets should be satisfied (Table 3).

**Table 3. Requirements during compression process**

| Parameters of tablets | Requirements |
|---|---|
| Appearance | round, biconvex, yellow, smooth tablets |
| Average mass of modified release layer | 280 mg (266 - 294 mg) |
| Average mass of immediate release layer | 110 mg (105 - 116 mg) |
| Average mass of tablets | 390 mg (371 - 410 mg) |
| Hardness of tablets | 60 - 130 N |
| Friability | NMT 1.0% |

For the pharmaceutical composition IR/1+MR/1, the above processes were used to prepare a batch of bilayer tablets using 46 kg of the granulate of the modified-release component and 18 kg of the granulate of immediate release component.

### Example 2. Dissolution studies

As prolonged-release pharmaceutical composition, a bilayer tablet having the composition of IR/1+MR/1 (in total 200 mg furazidin) was used. Dissolution testing was performed using the following conditions:

| | |
|---|---|
| Apparatus: | Baskets "1" |
| Media volume: | 1000 mL |
| Temperature: | 37.0°C ± 0.5°C |
| Speed: | 100 rpm |
| Sampling times: | 0.5, 1, 2, 4, 6, 8, 10, 12 and 14 hours |
| Medium: | phosphate buffer solution pH=6.8 with addition of 2.5% CTAB (hexadecyltrimethylammonium bromide) |

The quantities of drug substance released from the tested tablets are determined by HPLC. The mean value calculated from 6 tablets are indicated in Table 4.

**Table 4. Dissolution data (mean value calculated from 6 tablets)**

| Furazidin, prolonged-release tablets (IR/1+MR/1), 200 mg | | | |
|---|---|---|---|
| Time [h] | Mean [%] | SD | RSD |
| 0.5 | 18.3 | 3.0 | 16.4 |
| 1 | 22.1 | 4.0 | 18.0 |
| 2 | 27.6 | 3.6 | 13.2 |
| 4 | 37.7 | 3.7 | 9.9 |
| 6 | 48.9 | 3.3 | 6.7 |
| 8 | 60.3 | 3.1 | 5.1 |
| 10 | 71.4 | 3.5 | 4.9 |
| 12 | 93.2 | 3.4 | 3.7 |
| 14 | 93.3 | 2.8 | 3.0 |

The following Table 5 shows dissolution results of selected MR components. The MR components were pressed to tablets and subjected to a dissolution test using the above conditions. The sampling times are indicated in Table 5. The mean value calculated from 6 samples is indicated.

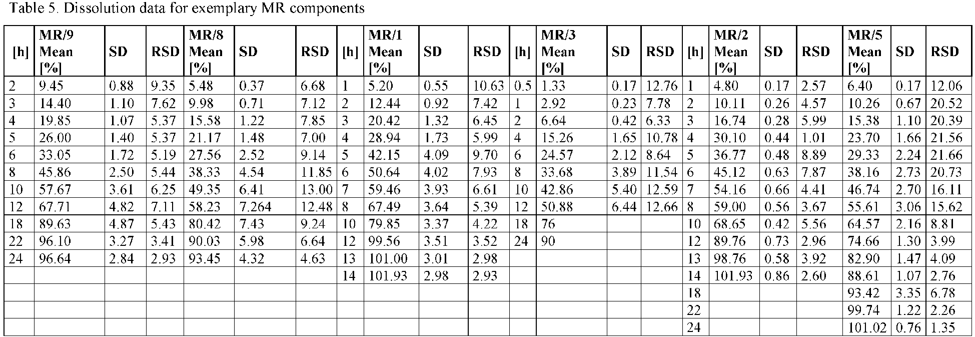

### Example 3. Swelling test - analysis of the MR matrix texture

In Example 3, the swelling properties of the MR component having a pH-independent polymer matrix and the dissolution of furazidin from the MR component were investigated. For the swelling test, the samples were prepared as follows. The test time and conditions are adjusted as to achieve complete dissolution of the IR layer and mimic the swelling characteristics of the MR component after 2 h mean retention time of the tested tablet in the stomach a after meal.

Sample preparation: Tablets, having the composition indicated in Table 6 below, were produced as described above in Example 1. The tablets (n=5) were placed in beaker filled with 30 to 35 mL of phosphate buffer of pH=4.5. The beaker is placed in wiggled, heated bath at a temperature of 37°C. The tablets are incubated for 2 hours.

The swollen samples were tested using a common texture analyser. Before testing, the probe of the texture analyser was calibrated against the testing platform (i.e. a blank slide).

After the IR component (if present) of the tablet was completely dissolved (completion after 2 h incubation), the swollen MR component (i.e. the MR component is the swollen state) of the tablets was transferred from the beaker on slides and singly tested.

For the data analysis, the values of interest for a sample were obtained by a macro included in the software of the TA.XTplus Texture Analyser. The macro quantifies the measured gel thickness. The average of 5 tablets was calculated. The results are summarized in Table 6 indicating the thickness of the MR component before swelling, the thickness range of the individual results after swelling, the average thickness after swelling and the swelling ratio (thickness before swelling/thickness in the swollen state).

The following settings were used:
Instrument/software: TA.XTplus Texture Analyser (Stable Micro Systems Ltd)/Exponent
Accessory: Load cell - 5 kg
Cylinder probe
TA settings:
   Mode: Measure Distance in Compression
   Pre-Test Speed: 1.0 mm/s
   Test speed: 0.1 mm/s
   Post-Test Speed: 5.0 mm/s
   Target Mode: Force

**Table 6. Thickness of the initial and swollen MR component and swelling ratio of the MR component after 2h incubation in phosphate buffer pH=4.5.**

| **Tablet composition (furazidin content)** | **pH-independent polymer** | **Thickness before swelling [mm]** | **Thickness range after swelling [mm]** | **Average thickness after swelling [mm]** | **Swelling ratio** |
|---|---|---|---|---|---|
| **IR/1+MR/7 (50 mg + 150 mg)** | hydroxypropylmethylcellulose, Methocel K4M | 3.6 | 4.7 - 5.7 | 5.3 | 1.45 |
| | hydroxyethylcellulose, Natrosol 6.2% | | | | |
| **IR/1+MR/1 (50 mg + 150 mg)** | hydroxypropylmethylcellulose, Methocel K4M | 3.6 | 4.9 - 6.2 | 5.4 | 1.5 |
| | hydroxyethylcellulose, Natrosol 8.2% | | | | |
| **IR/1+MR/8 (50 mg + 150 mg)** | hydroxypropylmethylcellulose, Methocel K4M | 3.6 | 4.3 - 5.3 | 4.9 | 1.6 |
| | poly(ethylene oxide), Polyox 7 000 000 | | | | |
| **IR/1+MR/9 (50 mg + 130 mg)** | hydroxypropylmethylcellulose Methocel K4M | 3.7 | 5.1 - 5.7 | 5.5 | 1.48 |
| | poly(ethylene oxide), Polyox 7 000 000 | | | | |
| **MR/3 (150 mg)** | hydroxypropylmethylcellulose, Methocel K4M hydroxypropylcellulose, Klucel LF | 3.8 | 4.4 - 4.8 | 4.6 | 1.2 |
| **MR/4 (150 mg)** | hydroxypropylmethylcellulose, Methocel K4M | 3.75 | 5.8 - 6.1 | 6.0 | 1.6 |
| | sodium carboxymethylcellulose, Blanose 7M31F | | | | |
| **MR/2 (150mg)** | hydroxypropylmethylcellulose, Methocel K4M | 3.7 | 5.8 - 7.0 | 6.6 | 1.78 |
| | xantan gum, Xantural 75 | | | | |
| **MR/6 (150 mg)** | hydroxypropylmethylcellulose, Methocel K15M | 3.9 | 4.9 - 5.7 | 5.3 | 1.35 |
| | sodium carboxymethylcellulose, Blanose 7M31F | | | | |
| **MR/5 (150 mg)** | hydroxypropylmethylcellulose, Methocel K15M xantan gum, Xantural 75 | 3.66 | 3.8 - 4.3 | 4.1 | 1.12 |

The dissolution amount of furazidin has been determined according to the dissolution test in Example 2. The mean values for several tablets are indicated in Table 7.

**Table 7. Dissolution amount of furazidin after 2 h under the conditions of Example 2.**

| **Tablet composition** | **Dissolution of furazidin Mean [%] after 2h** |
|---|---|
| IR/1+MR/7 | 24.2 - 30.5 |
| | 25.6 |
| | 28.3 |
| | 25.3 |
| | 26.0 |
| IR/1+MR/1 | 26.1 |
| | 24.1 |
| | 26.2 |
| IR/1+MR/8 | 23.7 |
| | 20.7 |
| IR/1+MR/9 | 30.7 |
| | 25.1 |
| | 28.2 |
| MR/3 | 6.64 |

It was found that the thickness of the swollen MR component correlated with the dissolution profile of tablets composed of IR component and MR component as well as of the dissolution profile of the MR component alone.

The 2h time point has been chosen as representative for the correlation and referent for the prediction of the required full dissolution profile. The dissolution values at the representative 2h time point showed a release of furazidin on the level of about 20 to 30 wt.% (a range of 10 to 35 wt.% is acceptable for a prolonged-release composition) for the bilayer tablets composed of IR component and MR component and a release of furazidin on the level of about 7 wt.% (a range of 5 to 15 wt.% was considered acceptable) for the MR component MR/3 when tested alone. It was found that the results are consistent with the expected levels of released furazidin over time to provide appropriate PK values in vivo.

### Example 4. Stability studies

Medicines must comply with the requirements of the chemical purity immediately after preparation (comply with the limits indicated in the release specification of the drug product), and within the period set by the relevant guidelines in time and storage conditions (comply with the limits indicated in the shelf-life specification of the drug product). The stability studies have been performed according to guideline CPMP/ICH/2736/99-ICH Q1A (R2) Stability Testing of New Drug Substances and Products. The main impurity of furazidin is ACRO, i.e. (2E)-3-(5-nitro-2-furylo)acrylaldehyde.

The final composition was packed in blisters, SBC 180/aluminum foil, and stored at long term (25°C/60% RH), intermediate (30°C/65% RH) and accelerated (40°C/75% RH) conditions. The level of impurities and the content of active substance were determined by HPLC according to the method disclosed in Journal of Pharmaceutical and Biomedical Analysis, Vol. 129 (2016), 433-440. The results are presented in Tables 8, 9 and 10.

**Table 8. Stability under long term conditions (25°C, 60% RH).**

| Parameter | Furazidin, prolonged-release tablets, 200 mg (IR/1+MR/1) | | | | | |
|---|---|---|---|---|---|---|
| | Requirements¹ | start | 3 months | 6 months | 9 months | 12 months |
| **Chromatographic purity:** | | | | | | |
| - impurity ACRO | not more than 0.1% | n.d. | n.d. | 0.06% | n.d. | n.d. |
| - single unspecified impurity | each not more than 0.2% | 0.05% | <0.05% | <0.05% | 0.03% | 0.03% |
| - total impurities | not more than 1.0% | 0.10% | <0.05% | 0.08% | 0.03% | 0.03% |
| **Content of the active substance in one tablet:** | 200.0 mg ± 5% (190.0 - 210.0 mg) | 198.9 mg | 202.0 mg | 199.7 mg | 203.6 mg | 204.2 mg |
| **Dissolution:** | | | | | | |
| - 1 hour | 10-30 % | 22% | 24% | 20% | 23% | 23% |
| - 6 hours | 40-65 % | 48% | 56% | 58% | 48% | 56% |
| - 14 hours | more than 80 % | 92% | 97% | 103% | 96% | 97% |

| | | | | | | |
|---|---|---|---|---|---|---|
| ¹The requirements for the parameters tested during the stability study were established according to guidelines CPMP/ICH/367/96 (ICH Q6A), and CPMP/ICH/2738/99 (ICH Q3B (R2)). | | | | | | |

**Table 9. Stability under intermediate conditions (30°C, 65% RH).**

| Parameter | Furazidin, prolonged-release tablets, 200 mg (IR/1+MR/1) | | | | | |
|---|---|---|---|---|---|---|
| | Requirements¹ | start | 3 months | 6 months | 9 months | 12 months |
| **Chromatographic purity:** | | | | | | |
| - impurity ACRO | not more than 0.1% | n.d. | n.d. | 0.06% | n.d. | n.d. |
| - single unspecified impurity | each not more than 0.2% | 0.05% | <0.05% | <0.05% | 0.03% | 0.03% |
| - total impurities | not more than 1.0% | 0.10% | <0.05% | 0.08% | 0.03% | 0.03% |
| **Content of the active substance in one tablet:** | 200.0 mg ± 5% (190.0 - 210.0 mg) | 198.9 mg | 200.3 mg | 200.8 mg | 206.6 mg | 203.9 mg |
| **Dissolution:** | | | | | | |
| - 1 hour | 10-30 % | 22% | 19% | 20% | 23% | 25% |
| - 6 hours | 40-65 % | 48% | 48% | 55% | 49% | 52% |
| - 14 hours | more than 80 % | 92% | 93% | 98% | 97% | 97% |

| | | | | | | |
|---|---|---|---|---|---|---|
| ¹The requirements for the parameters tested during the stability study were established according to guidelines CPMP/ICH/367/96 (ICH Q6A), and CPMP/ICH/2738/99 (ICH Q3B (R2)). | | | | | | |

**Table 10. Stability under enhanced conditions (40°C, 75% RH).**

| Parameter | Furazidin, prolonged-release tablets, 200 mg (IR/1+MR/1 | | | |
|---|---|---|---|---|
| | Requirements¹ | start | 3 months | 6 months |
| **Chromatographic purity:** | | | | |
| - impurity ACRO | not more than 0.1% | n.d. | n.d. | 0.04% |
| - single unspecified impurity | each not more than 0.2% | 0.05% | <0.05% | <0.05% |
| - total impurities | not more than 1.0% | 0.10% | <0.05% | 0.05% |
| **Content of the active substance in one tablet:** | 200.0 mg ± 5% (190.0 - 210.0 mg) | 198.9 mg | 203.0 mg | 204.6 mg |
| **Dissolution:** | | | | |
| - 1 hour | 10-30% | 22% | 24% | 20% |
| - 6 hours | 40-65% | 48% | 51% | 48% |
| - 14 hours | more than 80% | 92% | 95% | 95% |

| | | | | |
|---|---|---|---|---|
| ¹The requirements for the parameters tested during the stability study were established according to guidelines CPMP/ICH/367/96 (ICH Q6A), and CPMP/ICH/2738/99 (ICH Q3B (R2)). | | | | |

### Example 5. Phase I Clinical Trial Pivotal food effect study FUR-PK-03-19 (755/19)

**Study title:** Randomized, open-label, three-period, cross-over, food effect pharmacokinetic study comparing bioavailability of furazidin, prolonged-release tablets, 200 mg having a composition of IR/1+MR/1 according to Example 1 in healthy subjects in different fed conditions.

**Aim of the study:** The main study objective was to evaluate the pharmacokinetic properties and to compare the bioavailability of the Test product in healthy volunteers under different fed conditions.

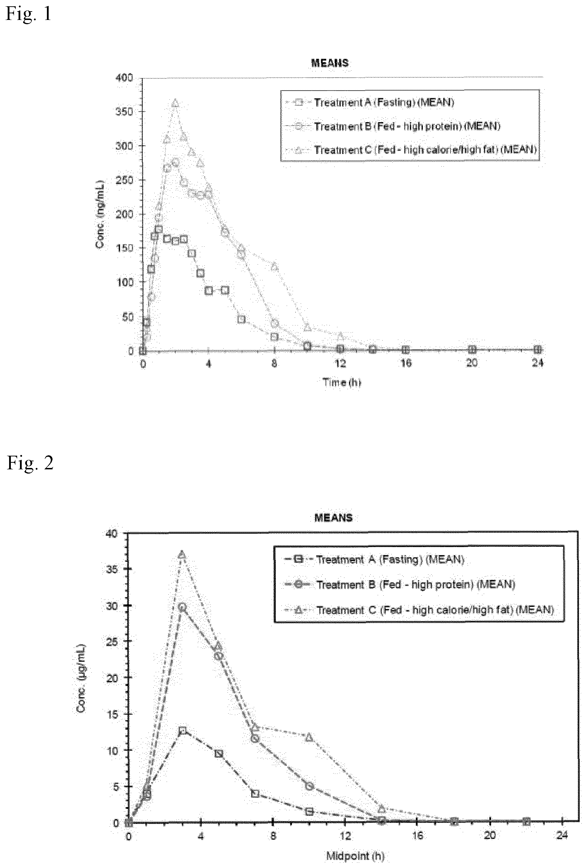

**Study conduct:** In this bioavailability study, the pharmacokinetic profile was tested for the Test formulation (prolonged-release bilayer tablet, IR/1+MR/1) having a strength of 200 mg furazidin. The bioavailability study was carried out under different fed conditions (after a high-protein meal or after a high-fat, high-calorie meal) and in fasting state in 30 healthy subjects. On the day of dosing, the subjects received randomly after an overnight fast a single dose of the modified-release formulation, administered on empty stomach (fasting conditions, Treatment A) or after a high-protein meal (Treatment B) or after a high-fat, high-calorie meal (Treatment C), each time with 240 mL of water. For each subject, there were 3 dosing periods, separated by a washout period of 7 days. Blood and urine samples were collected over 24 hours (20 blood samples and 9 urine samples). The results are shown in Tables 11 to 13 and Fig. 1 for the plasma analyses and in Tables 14 to 16 and Fig. 2 for the urine analyses.

The pharmacokinetic data for plasma and urine were statistically analysed in 29 subjects. After single oral administration of furazidin, prolonged-release formulation, 200 mg to the healthy volunteers, peak plasma concentrations were reached at 2 hours (median) following administration under recommended fed conditions (high-protein meal). Cₘₐₓ and AUC₀₋₂₄ reached 353.1±122.5 ng/mL and 1404.1±392.8 ng*h/mL, respectively. Plasma concentrations of furazidin showed a mean terminal half-life of 1.0 hour in healthy subjects. The mean urine concentrations of furazidin under recommended fed conditions achieved 9971.867± 3145.830 µg for Ae₀₋ₜ and 1933.965±841.315 µg/h for Rₘₐₓ. Peak urine concentrations were reached at 5 hours.

Total exposure (AUC) increases approximately 2 to 2.5-fold after high-protein and after high-fat, high calorie meal respectively compared to the fasting state, while Cₘₐₓ is increased to a lower extent. The exposure after a high fat, high calorie meal increases approximately 25% compared to the exposure after high-protein meal. Largest difference in exposure after meals versus fasting state is observed after 6 hours (AUC₍₆₋₂₄₎), which implies a difference in profile shape resulting from a much larger increase in exposure at later times post dosing. High-fat, high calorie meal increases the exposure from 6 to 24 hours about 5.6-fold. Coadministration of the modified-release formulation with a high-protein meal increases overall plasma and urine levels in the same way as it was observed for the immediate release product (Furaginum Adamed 50 mg tablets). Higher exposures observed after a high-fat, high-calorie meal do not appear related to dose dumping. Dose dumping related to formulation failure would result in higher Cₘₐₓ and exposures in the first four hours of the plasma concentration curves. Differences in profile shape between high-fat and high-protein administration are only observed in a subgroup of volunteers due to peaks occurring after 6 hours post dose, but it does not appear to be a formulation related issue because of the timing of differences.

**Safety Results:** Seven (7) subjects experienced a total of two (2) mild and seven (7) moderate adverse events (AE). In total, two adverse events were considered related to the oral administration of the Test product under fasting condition (Treatment A), i.e. one episode of mild nausea (and vomiting) and one episode of moderate pyrosis (heartburn). No adverse event was considered related to the oral administration of the Test product in fed condition, high-protein breakfast (Treatment B). One (1) adverse event was considered related to the oral administration of the Test product under fed condition, high-calorie/high-fat breakfast (Treatment C), i.e., one episode of moderate nausea (and vomiting). No serious adverse event (SAE) occurred during the Study.

**Table 11. Pharmacokinetic parameters in plasma of furazidin - high protein vs fasting conditions**

| **Treatment** | **AUC0-t (ng·h/mL)** | **AUC0-∞ (ng·h/mL)** | **AUC**_{**0**-}**₆ₕ (ng·h/mL)** | **AUC₆ₕ-t (ng·h/mL)** | **AUC₀₋₁₂ₕ (ng·h/mL)** | **Cₘₐₓ (ng/mL)** | **^{c}tₘₐₓ (h)** | **t_{1/2} (h)** |
|---|---|---|---|---|---|---|---|---|
| **^{a}Fasting** | 793.2± 387.0 | 801.9± 388.3 | 695.5± 260.6 | 97.7± 183.8 | 794.5± 380.1 | 256.7± 87.3 | 1.50 (0.8-5.0) | 1.09± 0.5 |
| **^{a}High protein** | 1404.1± 392.8 | 1415.1± 388.8 | 1168.5± 338.6 | 235.6± 168.2 | 1405.8± 391.5 | 353.1± 122.5 | 2.0 (0.8-6.0) | 1.02± 0.5 |
| **^{b}Ratio High protein vs Fasting (90% CL)** | 1.90 (1.69-2.14) | 1.89 (1.68-2.13) | 1.74 (1.57-1.92) | 3.59 (2.22-5.82) | 1.90 (1.6-2.13) | 1.39 (1.25-1.55) | - | - |
| **CVres High protein vs Fasting (%)** | 27.0 | 26.7 | 22.9 | 116.9 | 27.0 | 25.0 | - | - |

| | |
|---|---|
| **AUC₀₋ₜ** | area under the plasma concentration-time curve calculated from time zero until the last measurable plasma concentration |
| **AUC**_{**0**-**∞**} | area under the plasma concentration-time curve from time zero to infinity |
| **AUC**_{**0**-**6h**} | area under the plasma concentration-time curve from time zero to 6 hours |
| **AUC**_{**6h**-**t**} | area under the plasma concentration-time curve from time 6h to last measurable plasma concentration |
| **AUC**_{**0**-**12**} | area under the plasma concentration-time curve from time zero to 12 hours |
| **Cₘₐₓ** | maximum plasma concentration |
| **tₘₐₓ** | time for maximum concentration |
| **t_{1/2}** | half-life |
| **a** | arithmetic mean ± SD |
| **b** | In-transformed values |
| **c** | median, range |

**Table 12. Pharmacokinetic parameters in plasma of furazidin - high calorie/high fat vs fasting conditions**

| **Treatment** | **AUC(0-t) (ng·h/mL)** | **AUC**_{**(0-**∞}**₎ (ng·h/mL)** | **AUC₍₀₋₆ₕ₎ (ng·h/mL)** | **AUC₍₆ₕ₋ₜ₎ (ng·h/mL)** | **AUC₍₀₋₁₂ₕ₎ (ng·h/mL)** | **Cmax (ng/mL)** | **^{c}tₘₐₓ (h)** | **t_{1/2} (h)** |
|---|---|---|---|---|---|---|---|---|
| **^{a}Fasting** | 793.2± 387.0 | 801.9± 388.3 | 695.5± 260.6 | 97.7± 183.8 | 794.5± 380.1 | 256.7± 87.3 | 1.50 (0.8-5.0) | 1.1± 0.5 |
| **^{a}High calorie/ High Fat** | 1885.0± 578.1 | 1869.6± 574.6 | 1374.1± 498.6 | 510.9± 503.1 | 1861.7± 561.2 | 433.3± 147.0 | 2.0 (1.0-12.0) | 0.9± 0.2 |
| **^{b}Ratio High calorie/ High fat vs Fasting (90% CL)** | 2.52 (2.23-2.85) | 2.46 (2.17-2.78) | 2.0 (1.73-2.30) | 563.5 (3.30-9.62) | 2.49 (2.20-2.80) | 1.72 (1.51-1.95) | - | - |
| **CVres High calorie/ High fat vs Fasting (%)** | 27.8 | 27.5 | 32.6 | 27.3 | 27.3 | 29.7 | - | - |

| | |
|---|---|
| **AUC₀₋ₜ** | area under the plasma concentration-time curve calculated from time zero until the last measurable plasma concentration |
| **AUC_{0-∞}** | area under the plasma concentration-time curve from time zero to infinity |
| **AUC₀₋₆ₕ** | area under the plasma concentration-time curve from time zero to 6 hours |
| **AUC**_{**6h**-**t**} | area under the plasma concentration-time curve from time 6h to last measurable plasma concentration |
| **AUC₀₋₁₂** | area under the plasma concentration-time curve from time zero to 12 hours |
| **Cₘₐₓ** | maximum plasma concentration |
| **tₘₐₓ** | time for maximum concentration |
| **t_{1/2}** | half-life |
| **a** | arithmetic mean ± SD |
| **b** | In-transformed values |
| **c** | median, range |

**Table 13. Pharmacokinetic parameters in plasma of furazidin - high protein vs high calorie/high fat conditions**

| **Treatment** | **AUC₍₀₋ₜ₎ (ng·h/mL)** | **AUC_{(0-∞)} (ng·h/mL)** | **AUC₍₀₋₆ₕ₎ (ng·h/mL)** | **AUC₍₆ₕ₋ₜ₎ (ng·h/mL)** | **AUC₍₀₋₁₂ₕ₎ (ng·h/mL)** | **Cmax (ng/mL)** | **^{c}tₘₐₓ (h)** | **t_{1/2} (h)** |
|---|---|---|---|---|---|---|---|---|
| **^{a}High protein** | 1404.1± 392.8 | 1415.1± 388.8 | 1168.5± 338.6 | 235.6± 168.2 | 1405.8± 391.5 | 353.1± 122.5 | 2.0 (0.8-6.0) | **1.0** ± 0.5 |
| **^{a}High calorie/ High fat** | 1885.0± 578.1 | 1869.6± 574.6 | 1374.1± 498.6 | 510.9± 503.1 | 1861.7± 561.2 | 433.3± 147.0 | 2.0 (1.0-12.0) | 0.9 ± 0.2 |
| **^{b}Ratio High protein vs High calorie/High fat (90% CL)** | 0.75 (0.69-0.82) | 0.77 (0.71-0.83) | 0.87 (0.78-0.97) | 0.59 (0.43-0.80) | 0.76 (0.70-0.83) | 0.81 (0.74-0.90) | - | - |
| **CVres High protein vs High calorie/High fat (%)** | 18.7 | 17.9 | 25.1 | 76.6 | 17.8 | 22.1 | - | - |

| | |
|---|---|
| **AUC₀₋ₜ** | area under the plasma concentration-time curve calculated from time zero until the last measurable plasma concentration |
| **AUC_{0-∞}** | area under the plasma concentration-time curve from time zero to infinity |
| **AUC₀₋₆ₕ** | area under the plasma concentration-time curve from time zero to 6 hours |
| **AUC₆ₕ₋ₜ** | area under the plasma concentration-time curve from time 6h to last measurable plasma concentration |
| **AUC₀₋₁₂** | area under the plasma concentration-time curve from time zero to 12 hours |
| **Cₘₐₓ** | maximum plasma concentration |
| **tₘₐₓ** | time for maximum concentration |
| **t_{1/2}** | half-life |
| **a** | arithmetic mean ± SD |
| **b** | In-transformed values |
| **c** | median, range |

Fig. 1 shows the pharmacokinetic profiles of means (N=29) in plasma under fasting conditions (Treatment A), under fed conditions of a high-protein breakfast (Treatment B) and under fed conditions of a high-calorie/high-fat breakfast (Treatment C).

**Table 14. Pharmacokinetic parameters in urine of furazidin - high protein vs fasting conditions**

| **Treatment** | **AURC₍₀₋ₜ₎ (µg)** | **AURC_{(0-∞)} (µg)** | **Ae₍₀₋ₜ₎ (µg)** | **Rmax (µg/h)** | **^{c}tₘₐₓ (h)** | **t_{1/2} (h)** |
|---|---|---|---|---|---|---|
| **^{a}Fasting** | 5653.5± 3649.3 | 6519.2± 4484.2 | 5786.3± 3421.2 | 1399.7± 674.1 | 2.7 (2.0-7.0) | 2.6± 3.5 |
| **^{a}High protein** | 9858.8± 3121.1 | 9579.0± 3460.9 | 9971.9± 3145.8 | 1934.0± 841.3 | 4.6 (2.2-10.0) | 1.6± 0.9 |
| **^{b}Ratio High protein vs Fasting (90% CL)** | 1.90 (1.55-2.32) | 1.27 (0.79-2.0) | 1.84 (1.51-2.24) | 1.39 (1.16-1.65) | - | - |
| **CVres High protein vs Fasting (%)** | 47.5 | 59.6 | 45.8 | 41.0 | - | - |

| | |
|---|---|
| **AURC₍₀₋ₜ₎** | Area under the urine excretion rate-time curve to the last measurable excretion rate |
| **AURC_{(0-∞)}** | Area under the urine excretion rate-time curve extrapolated to infinity, based on the last observed excretion rate |
| **Ae₀₋ₜ** | amount of unchanged drug excreted into the urine calculated as area under the urinary excretion rate curve from time zero to the last measurable rate |
| **Rₘₐₓ** | the maximum rate of drug excretion observed |
| **tₘₐₓ** | time of maximum urinary excretion rate |
| **t_{1/2}** | half-life |
| **a** | arithmetic mean ± SD |
| **b** | In-transformed values |
| **c** | median, range |

**Table 15. Pharmacokinetic parameters in urine of furazidin - high calorie/high fat vs fasting conditions**

| **Treatment** | **AURC₍₀₋ₜ₎ (µg)** | **AURC_{(0-∞)} (µg)** | **Ae₍₀₋ₜ₎ (µg)** | **Rmax (µg/h)** | **^{c}tₘₐₓ (h)** | **t_{1/2} (h)** |
|---|---|---|---|---|---|---|
| **Fasting** | 5653.5± 3649.3 | 6519.2± 4484.2 | 5786.3± 3421.2 | 1399.7± 674.1 | 2.7 (2.0-7.0) | 2.6± 3.5 |
| **High calorie/High fat** | 13126.5± 5026.2 | 16959.3± 12255.5 | 13791.1± 5445.0 | 2197.4± 858.4 | 2.9 (2.0-13.8) | 3.06± 5.03 |
| **Ratio High calorie/ High fat vs Fasting (90% CL)** | 244.6 (209.8-285.3) | 200.8 (115.4-349.3) | 245.6 (212.7-283.5) | 159.4 (139.6-182.0) | - | - |
| **CVres High calorie/ High fat vs Fasting (%)** | 35.3 | 62.4 | 32.8 | 30.2 | - | - |

| | |
|---|---|
| **AURC₍₀₋ₜ₎** | Area under the urine excretion rate-time curve to the last measurable excretion rate |
| **AURC_{(0-∞)}** | Area under the urine excretion rate-time curve extrapolated to infinity, based on the last observed excretion rate |
| **Ae₀₋ₜ** | amount of unchanged drug excreted into the urine calculated as area under the urinary excretion rate curve from time zero to the last measurable rate |
| **Rₘₐₓ** | the maximum rate of drug excretion observed |
| **tₘₐₓ** | time of maximum urinary excretion rate |
| **t_{1/2}** | half-life |
| **a** | arithmetic mean ± SD |
| **b** | In-transformed values |
| **c** | median, range |

**Table 16. Pharmacokinetic parameters in urine of furazidin - high protein vs high calorie/high fat conditions**

| **Treatment** | **AURC₍₀₋ₜ₎ (µg)** | **AURC_{(0-∞)} (µg)** | **Ae₍₀₋ₜ₎ (µg)** | **Rmax (µg/h)** | **^{c}tₘₐₓ (h)** | **t_{1/2} (h)** |
|---|---|---|---|---|---|---|
| **High protein** | 9858.8± 3121.1 | 9579.0± 3460.9 | 9971.9± 3145.8 | 1934.0± 841.3 | 4.6 (2.2-10.0) | 1.6± 0.9 |
| **High calorie/High fat** | 13126.5± 5026.2 | 16959.3± 12255.5 | 13791.1± 5445.0 | 2197.4± 858.4 | 2.9 (2.0-13.8) | 3.06± 5.03 |
| **Ratio High protein vs High calorie/High fat (90% CL)** | 77.5 (66.9-89.8) | 71.9 (52.2-98.9) | 75.0 (64.6-87.0) | 87.0 (75.0-100.8) | - | - |
| **CVres High protein vs High calorie/High fat (%)** | 33.6 | 38.8 | 34.0 | 33.8 | - | - |

| | |
|---|---|
| **AURC₍₀₋ₜ₎** | Area under the urine excretion rate-time curve to the last measurable excretion rate |
| **AURC_{(0-∞)}** | Area under the urine excretion rate-time curve extrapolated to infinity, based on the last observed excretion rate |
| **Ae₀₋ₜ** | amount of unchanged drug excreted into the urine calculated as area under the urinary excretion rate curve from time zero to the last measurable rate |
| **Rₘₐₓ** | the maximum rate of drug excretion observed |
| **tₘₐₓ** | time of maximum urinary excretion rate |
| **t_{1/2}** | half-life |
| **a** | arithmetic mean ± SD |
| **b** | In-transformed values |
| **c** | median, range |

Fig. 2 shows the pharmacokinetic profiles of means (N=29) in urine under fasting conditions (Treatment A), under fed conditions of a high-protein breakfast (Treatment B) and under fed conditions of a high-calorie/high-fat breakfast (Treatment C).

### Conclusions:

- Bioavailability was found to be 1.9-fold higher after high-protein meal and 2.5-fold higher after high-calorie/high-fat meal than under fasting conditions, (i.e. bioavailability after high-protein meal is about 25% lower than after high-calorie/high-fat meal).
- Peak plasma concentration was found to be 1.4-fold higher after high-protein meal and 1.7-fold higher after high calorie/high-fat meal than under fasting conditions, (i.e. peak plasma concentration after high-protein meal is about 19% lower than after high-calorie/high-fat meal).
- Excreted amount of unchanged drug was found 1.9-fold higher after high-protein meal and 2.5-fold higher after high-calorie/high-fat meal than under fasting conditions, (i.e. excreted amount of unchanged drug after high-protein meal is about 25% lower than after high-calorie/high-fat meal).
- Urine excretion rate was found 1.4-fold higher after high-protein meal and 1.6-fold higher after high-calorie/high-fat meal than under fasting conditions, (i.e. urine excretion rate after high-protein meal is about 13% lower than after high-calorie/high-fat meal).
- The 90% confidence intervals for fed1*/fast, fed2*/fast and fed1*/fed2* ratios of LSM, based on ln-transformed data of AUC₍₀₋ₜ₎ and Cₘₐₓ were not found within the equivalence limits of 80.00-125.00 %. Hence, a significant food effect was confirmed.
- In general, a 25% increase in exposure after high-fat, high-calorie meal should not translate to a different safety profile, unless the drug has a narrow therapeutic index, which is not the case for the well-established drug substance furazidin.
- Furazidin administered orally should be taken at or after meals as this increases its bioavailability irrespective of the formulation or mechanisms of the drug release (MR or IR). Differences in concentrations after high-protein meal and after high-fat, high-calorie meal are statistically significant.

### Example 6. Pivotal multiple-dose study FUR-PK-04-19 (759/19)

**Study title:** Randomized, open-label, two-period, multiple dose, cross-over, pivotal pharmacokinetic study comparing bioavailability of Furazidin, prolonged-release tablets, 200 mg of the composition IR/1+MR/1 according to Example 1 and Furaginum Adamed 50 mg, tablets in healthy male and female subjects under fed (high-protein meal) conditions.

**Aim of the study:** The main study objective was to evaluate the pharmacokinetic properties and to compare the bioavailability of furazidin after multiple-dose administration of the Test product versus the Comparator product in healthy male and female volunteers under fed (high-protein meal) conditions.

**Study conduct:** Thirty (30) subjects were included into the study. The elimination half-life of prolonged-release furazidin is about 1 hour. The elimination half-life reported in literature for immediate-release furazidin is no longer than 7 hours. In plasma, steady-state can be achieved within 1 to 2 days of twice-daily dosing of 200 mg prolonged-release furazidin tablets according to the present invention and three times daily dosing of 100 mg immediate-release furazidin tablets, respectively, so as to demonstrate that steady state has been reached when five (5) doses of furazidin, prolonged-release tablet, 200 mg according to the present invention and seven (7) doses of Furaginum Adamed, 50 mg, tablets were administered orally on Day 1 through Day 3. Dosing on Day 3 was continued up to 6 doses in total of prolonged-release tablets and, in total, 9 doses of immediate-release tablets to compare the total exposure after multiple dose administration.

Multiple oral doses of the Test product (6 doses) administered orally with 240 mL of water under fed (high-protein meal) conditions. Oral administration of one tablet given two times a day (12 hours interval apart). Total dose per day was 400 mg of furazidin. Dosing for 3 consecutive days (on Day 1, Day 2 and Day 3 twice a day). Multiple oral dose of Comparator product (9 doses) administered orally with 240 mL of water under fed (high-protein meal) conditions.

Oral administration of two tablets given at the same time (2 x 50 mg) three times a day (8 hours interval apart). Total dose per day was 300 mg of furazidin. Dosing for 3 consecutive days (on Day 1, Day 2 and Day 3 three times a day).

The washout period was at least 7 days between the last dose in Period 1 and the first dose in Period 2. Blood samples were collected throughout a 76-hour period after the first IMP administration to characterize products absorption and elimination. Urine samples were collected over 24 hours post first dose on Day 1-Day 2 and over 28 hours post the first dose on Day 3-Day 4.

Twenty-nine (29) subjects completed the study, and the data was used for the statistical analysis. Pharmacokinetic parameters were calculated from plasma and urine concentrations determined by a validated HPLC/MS/MS method for furazidin according to the method disclosed in Journal of Pharmaceutical and Biomedical Analysis, Vol. 129 (2016), 433-440. Pharmacokinetic parameters of the Test and the Comparator products were compared. The 90% confidence intervals for the Test to Comparator ratios were evaluated for the following pharmacokinetic parameters, which were considered as primary for bioavailability assessment:
Day 1 doses PK metrics: AUC₀₋₂₄, Cₘₐₓ and AURC₀₋₂₄
Day 3 doses PK metrics: AUC_{0-24,ss} (to reflect safety) and AURC_{0-24,Day3} (to reflect efficacy).

Concentration-time profiles of C_{through} concentrations were provided to assess reaching of steady-state. High C_{through} variability was observed as usual for very low plasma concentration levels at time points beyond 5 times the plasma elimination half-life. This is not unexpected as the elimination half-life is approximately 1 hr and so, by the 12-hour interval, more than 95% of the absorbed dose is eliminated. In fact, the majority of subjects showed C_{through} levels below the limit of quantitation. Hence, a steady state based on similar C_{through} levels cannot be established as the pharmacokinetics of the product at the 12-hour dosage interval were essentially repeat single doses.

According to the Study Protocol, the AUC₀₋₂₄ₕ, Cₘₐₓ and AURC₀₋₂₄ₕ for Day 1 and AUC_{0-24h,ss} and AURC_{0-24h,Day3} for Day 3 for furazidin were used to assess equivalence between both tested formulations. The results confirm that the 90% confidence intervals for Test to Comparator ratios of the geometric least squares means for above mentioned PK parameters were not within the bioequivalence acceptance range of 80.00% to 125.00%.

The results further confirmed that the exposure to the Test formulation was higher in terms of extent and intensity than exposure to the Comparator formulation both in plasma and urine.

**Safety Results:** Seven (7) subjects experienced a total of sixteen (16) moderate adverse events (AE). In total, four (4) adverse events were considered related to the oral administration of the Test product (i.e. the episodes of moderate headache), and six (6) adverse events (i.e. also episodes of moderate headache) were considered related to the oral administration of Comparator product.

**Table 17. Pharmacokinetic parameters of furazidin in plasma after single dose administration (Day 1)**

| **Treatment** | **AUC₀₋₂₄ₕ (ng h/mL)** | **Cₘₐₓ (ng/mL)** | **^{c}tₘₐₓ (h)** | **t_{1/2} (h)** |
|---|---|---|---|---|
| **^{a}Test** | 3614.6±1132.1 | 562.7±188.1 | 3.00 (1.50-6.00) | 0.96±0.26 |
| **^{a}Comparator** | 2760.5±791.5 | 342.0±96.8 | 2.00 (1.00-5.00) | 1.05±0.80 |
| **^{b}Ratio (90% CL)** | 1.30 (1.16-1.45) | 1.63 (1.40-1.89) | - | - |
| **Intra CV** | 25.59 | 34.30 | - | - |

| | |
|---|---|
| **AUC₀₋₂₄ₕ** | The area under the plasma concentration-time curve calculated from time zero until 24 hours |
| **Cₘₐₓ** | The maximum plasma concentration observed (ng/mL) |
| **tₘₐₓ** | The time of maximum plasma drug concentration (h) |
| **t_{1/2}** | The elimination half-life associated with the terminal slope of a semi-logarithmic plasma concentration-time curve or the urine excretion rate-time curve (h), t_{1/2}= ln(2)/λz |
| **a** | arithmetic mean ± SD |
| **b** | In-transformed values |
| **c** | median, range |

**Table 18. Pharmacokinetic parameters of furazidin in plasma after multiple-dose dose administration (Day 3)**

| **Treatment** | **AUC_{0-24h,ss} (ng h/ml)** | **C_{max,ss} (ng/ml)** | **C_{min,ss} (ng/ml)** | **Fluctuation (%)** | **Accumulation ratio AUC_{0-τ,ss}/ AUC_{0-τ}** | **Accumulation ratio C_{max,ss}/Cₘₐₓ** | **^{c}t_{max,ss} (h)** | **t_{1/2,ss} (h)** |
|---|---|---|---|---|---|---|---|---|
| **^{a}Test** | 3498.1 ±932.2 | 516.4 ±149.3 | 3.1 ±5.3 | 350.0 ±78.7 | 0.94 ±0.2 | 0.99 ±0.3 | 51.00 (49.02-53.00) | 1.05 ±0.39 |
| **^{a}Compara tor** | 2828.2 ±730.1 | 350.1± 93.1 | 11.6 ±19.2 | 266.5 ±62.7 | 1.0 ±0.5 | 1.1 ±0.4 | 50.00 (48.75-53.07) | 1.00 ±0.41 |
| **^{b}Ratio (90% CL)** | 1.24 (1.17-1.31) | 1.47 (1.33-1.62) | - | - | - | - | - | - |
| **Intra CV** | 13.34 | 22.44 | - | - | - | - | - | - |

| | |
|---|---|
| **AUC**_{**0**-**24h,ss**} | The area under the plasma concentration-time curve calculated from time zero until 24 hours |
| **C_{max,ss}** | The maximum plasma concentration observed (ng/mL) |
| **AUC**_{**0**-τ} | Area under the plasma concentration versus time curve (ng h/mL) calculated by the linear trapezoidal rule from sampling time zero to sampling time τ = 12 h (Treatment A) or τ = 8 h (Treatment B). |
| **AUC_{0-τ,ss}** | Area under the plasma concentration versus time curve (ng h/mL) calculated by the linear trapezoidal rule from sampling time zero to sampling time τ = 12 h (Treatment A) or τ = 8 h (Treatment B) in steady-state. |
| **t_{max,ss}** | The time of maximum plasma drug concentration (h) |
| **t_{1/2,ss}** | The elimination half-life associated with the terminal slope of a semi-logarithmic plasma concentration-time curve or the urine excretion rate-time curve (h), t_{1/2}= ln(2)/λz |
| **C_{min,ss}** | arithmetic mean ± SD |
| **a** | In-transformed values |
| **b** | median, range |
| **c** | median, range |

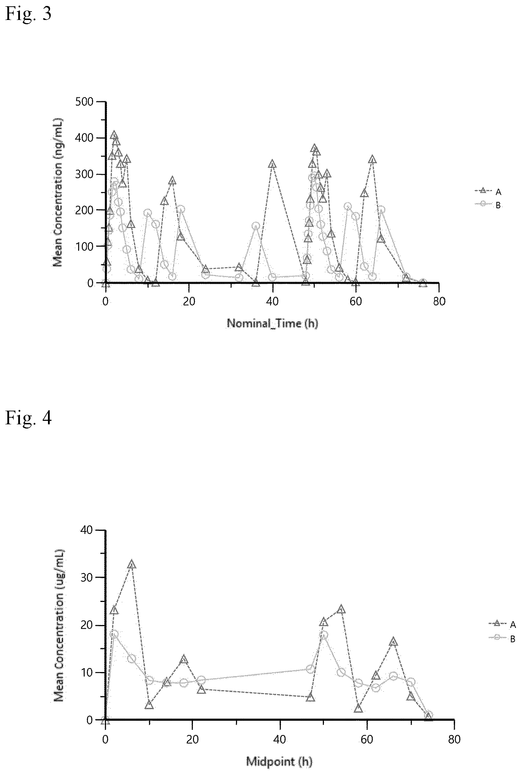

Fig. 3 shows the pharmacokinetic profile of means (N=29) in plasma (A=Test product, B=Comparator).

Fig. 4 shows the pharmacokinetic profile of means (N=29) in urine (A=Test product, B=Comparator).

**Table 19. Pharmacokinetic parameters of furazidin in urine after single and multiple-dose administration**

| **Treatment** | **AURC**_{**0**-}**₂₄ₕ (µg)** | **AURC_{0-24h,Day 3} (µg)** | **Ae₀₋₂₄ₕ (µg)** | **Ae**_{**0**-}**_{24h,Day3} (µg)** | **Rₘₐₓ (µg/h)** | **R_{max,Day3} (µg/h)** |
|---|---|---|---|---|---|---|
| **^{a}Test** | 21003.3 ±7904.2 | 24300.7 ±6549.63 | 23254.14 ±8718.06 | 25290.63 ±7010.8 | 2160.61 ±817.09 | 2415.47 ±897.17 |
| **^{a}Comparator** | 17864 ±6108.74 | 19520.3 ±5582.01 | 20158.81 ±6578.87 | 21066.96 ±6171.53 | 1566.51 ±569.18 | 1587.94 ±466.02 |
| **^{b}Ratio (90% CL)** | 1.20 (1.02-1.42) | 1.26 (1.14-1.39) | 1.16 (0.99-1.37) | 1.21 (1.10-1.34) | 1.39 (1.18-1.65) | 1.49 (1.29-1.73) |
| **Intra CV** | 38.5 | 22.5 | 37.5 | 22.01 | 38.8 | 32.9 |

| | |
|---|---|
| **AURC₀₋₂₄ₕ** | Area under the urine excretion rate-time curve from sampling time zero to time 24 h (µg) |
| **AURC_{0-24h,Day3}** | Area under the urine excretion rate-time curve from sampling time zero to time 24 h after multiple dose administration (µg) |
| **Ae₀₋₂₄ₕ** | Cumulative urinary excretion of unchanged drug from sampling time zero to time 24 h calculated as Sum of Volume x Concentration increments (µg) |
| **Ae_{0-24h,Day3}** | Cumulative urinary excretion of unchanged drug from sampling time zero to time 24 h calculated as Sum of Volume x Concentration increments after multiple dose administration (µg) |
| **Rₘₐₓ** | The maximum rate of drug excretion observed (µg/h) |
| **R_{max,Day3}** | the maximum urinary excretion rate observed after multiple dose administration (µg/h) |
| **a** | arithmetic mean ± SD |
| **b** | In-transformed values |

**Conclusion:** No accumulation was observed after multiple dosing and steady state was not confirmed for Test product after 3 days of dosing as expected by the short elimination half-life and the associated kinetics of repeat single doses. Bioavailability of Test product was higher after single and multiple dose administration compared to the Comparator product.

**Summary PK:** Furazidin is mainly absorbed in the upper part of the small intestine. Bioavailability was found 1.9-fold higher after high-protein meal than under fasting conditions. After single oral administration of the furazidin prolonged-release tablet, 200 mg according to the present invention to healthy volunteers in fed conditions (high-protein meal), the peak serum concentration 353.1±122.5 ng/mL (Cₘₐₓ) was reached within approx. 2.5 hour (tₘₐₓ), AUC₀₋₂₄ reached 1404.1±392.8 ng*h/mL. Following administration of the furazidin prolonged-release tablet 200 mg in a fasting state, the maximum serum concentration 256.7±87.3 ng/mL (Cₘₐₓ) was reached within 2 hours (tₘₐₓ). After multiple dose oral administration of furazidin prolonged-release tablet, 200 mg to the healthy volunteers for 3 days (twice a day, 12 hours apart), peak plasma concentrations were reached at about 51 hours following administration under fed conditions (high-protein meal). C_{max,ss} and AUC_{0-24,ss} reached 516.4±149.3 ng/mL and 3498.1±932.2 ng*h/mL, respectively.

## Claims

1. A prolonged-release pharmaceutical composition for oral administration, comprising:
a) an immediate release component, comprising furazidin and one or more pharmaceutically acceptable excipients; and
b) a modified-release component, comprising furazidin, a controlled-release agent, and one or more pharmaceutically acceptable excipients,
wherein
the pharmaceutical composition is a single dosage form in the form of a bilayer tablet, in which the immediate release component a) forms a first layer, the modified-release component b) forms a second layer, and the first layer at least partially covers the surface of the second layer,
in a matrix of the modified-release component b), the controlled-release agent comprises at least one pH-independent polymer, which is at least one selected from the group consisting of hydroxypropylmethylcellulose (HMPC), hydroxyethylcellulose (HEC), hydroxypropylcellulose, xanthan gum, sodium carboxymethylcellulose, non-ionic poly(ethylene oxide), and any combination thereof,
the immediate-release component has an in vitro dissolution rate of at least 80 wt.% of the furazidin within 45 minutes, based on the total furazidin content of the immediate release component,
the modified-release component has an in vitro dissolution rate of 5 to 15 wt.% of furazidin within 2 h, based on the total furazidin content of the modified-release component, and
the prolonged-release pharmaceutical composition has an in vitro dissolution rate of furazidin of 10 to 40 wt.% within 1 h, 40 to 65 wt.% within 6 h and above 80 wt.% within 14 h, based on the total furazidin content of the single dosage form, and
the dissolution rates being determined by using European Pharmacopoeia edition 10 Apparatus Baskets "1" in 1000 ml volume of phosphate buffer solution of pH=6.8 with the addition of 2.5% CTAB (hexadecyltrimethylammonium bromide) at 37.0°C±0.5°C and 100 rpm.

2. The pharmaceutical composition according to claim 1, wherein the immediate release component a) is contained as granulates, and the modified-release component b) is contained as granulates,
wherein the immediate release component a) and/or the modified-release component b) optionally also contain an extragranular fraction comprising one or more of a lubricant, a filler, an absorbent and a glidant.

3. The pharmaceutical composition according to claim 1 or 2, wherein the pharmaceutical composition comprises, in total, 150 to 250 mg of furazidin, preferably 180 to 220 mg of furazidin, and more preferably 200 mg of furazidin.

4. The pharmaceutical composition according to claim 3, wherein the immediate release component a) comprises 35 to 55 mg of furazidin, and the modified-release component b) comprises 145 to 165 mg of furazidin; and, preferably, the immediate release component a) comprises 45 to 55 mg of furazidin, and the modified-release component b) comprises 145 to 155 mg of furazidin; and, more preferably, the immediate release component a) comprises 50 mg of furazidin, and the modified-release component b) comprises 150 mg of furazidin.

5. The pharmaceutical composition according to any one of claims 1 to 4, wherein the modified-release component b) comprises 10 to 40 wt.% of the controlled-release agent, preferably 10 to 35 wt.% and more preferably 20 to 30 wt.% of the controlled-release agent, based on the total weight of the modified-release component b).

6. The pharmaceutical composition according to any one of claims 1 to 5, wherein the immediate release component a) comprises, as the at least one excipient, at least one of a filler, a disintegrant, a binder, a lubricant, a glidant and any combination thereof,
preferably, the immediate release component a) comprises 10 to 50 wt.% of a filler, 0.5 to 6 wt.% of a disintegrant, 1.4 to 1.7 wt.% of a glidant, 2 to 4 wt.% of a lubricant, and, optionally, 0.4 to 5 wt.% of a binder, based on the total weight of the immediate release component a).

7. The pharmaceutical composition according to any one of claims 1 to 6, wherein the modified-release component b) comprises, as the at least one excipient, at least one of a filler, a disintegrant, a binder, a lubricant, a glidant and any combination thereof,
preferably, the modified-release component b) comprises 10 to 40 wt.% of the controlled-release agent and 10 to 45 wt.% of a filler, 0.3 to 5 wt.% of a disintegrant, 0.4 to 5 wt.% of a binder, 0.5 to 2 wt.% of a lubricant, and, optionally, up to 1.3 wt.% of a glidant, based on the total weight of the modified-release component b).

8. The pharmaceutical composition according to any one of claims 1 to 7, wherein the pharmaceutical composition has an in vitro dissolution rate of furazidin of 10 to 30 % within 1 h.

9. A pharmaceutical composition as defined in any one of claims 1 to 8 for use in a method of treatment of a urinary tract infection.

10. The pharmaceutical composition for use according to claim 9, wherein the urinary tract infection is an acute or recurrent uncomplicated urinary tract infection.

11. The pharmaceutical composition for use according to claim 9 or 10, wherein the pharmaceutical composition is a single dosage form, which is administered orally two times daily, and preferably 11 to 13 hours apart, and more preferably 12 hours apart.

## Patentansprüche

1. Pharmazeutische Zusammensetzung mit verlängerter Freisetzung zur oralen Verabreichung, umfassend:
a) eine Komponente mit sofortiger Freisetzung, umfassend Furazidin und einen oder mehrere pharmazeutisch annehmbare Hilfsstoffe; und
b) eine Komponente mit modifizierter Freisetzung, umfassend Furazidin, ein Mittel zur kontrollierten Freisetzung und einen oder mehrere pharmazeutisch annehmbare Hilfsstoffe,
wobei
die pharmazeutische Zusammensetzung eine Einzeldosierungsform in Form einer Zweischichttablette ist, in der die Komponente mit sofortiger Freisetzung a) eine erste Schicht bildet, die Komponente mit modifizierter Freisetzung b) eine zweite Schicht bildet und die erste Schicht die Oberfläche der zweiten Schicht zumindest teilweise bedeckt,
in einer Matrix der Komponente mit modifizierter Freisetzung b) das Mittel zur kontrollierten Freisetzung mindestens ein pH-unabhängiges Polymer umfasst, das mindestens eines ist, das aus der Gruppe, bestehend aus Hydroxypropylmethylcellulose (HMPC), Hydroxyethylcellulose (HEC), Hydroxypropylcellulose, Xanthangummi, Natriumcarboxymethylcellulose, nichtionischem Poly(ethylenoxid) und einer beliebigen Kombination davon, ausgewählt ist,
die Komponente mit sofortiger Freisetzung eine In-vitro-Auflösungsrate von mindestens 80 Gew.-% des Furazidins innerhalb von 45 Minuten, basierend auf dem Gesamtfurazidingehalt der Komponente mit sofortiger Freisetzung, aufweist,
die Komponente mit modifizierter Freisetzung eine In-vitro-Auflösungsrate von 5 bis 15 Gew.-% des Furazidins innerhalb von 2 h, basierend auf dem Gesamtfurazidingehalt der Komponente mit modifizierter Freisetzung, aufweist, und
die pharmazeutische Zusammensetzung mit verlängerter Freisetzung eine In-vitro-Auflösungsrate von Furazidin von 10 bis 40 Gew.-% innerhalb von 1 h, 40 bis 65 Gew.-% innerhalb von 6 h und über 80 Gew.-% innerhalb von 14 h, basierend auf dem Gesamtfurazidingehalt der Einzeldosierungsform, aufweist, und
die Auflösungsraten unter Verwendung der Apparatur Körbe "1" der European Pharmacopoeia, 10. Ausgabe, in 1000 ml Volumen einer Phosphatpufferlösung mit pH = 6,8 mit Zugabe von 2,5% CTAB (Hexadecyltrimethylammoniumbromid) bei 37,0°C ± 0,5°C und 100 U/min bestimmt werden.

2. Pharmazeutische Zusammensetzung gemäß Anspruch 1, wobei die Komponente mit sofortiger Freisetzung a) als Granulat enthalten ist und die Komponente mit modifizierter Freisetzung b) als Granulat enthalten ist,
wobei die Komponente mit sofortiger Freisetzung a) und/oder die Komponente mit modifizierter Freisetzung b) optional auch eine extragranulare Fraktion enthält, die eines oder mehrere von einem Gleitmittel, einem Füllstoff, einem Absorptionsmittel und einem Fließregulierungsmittel (Glidant) umfasst.

3. Pharmazeutische Zusammensetzung gemäß Anspruch 1 oder 2, wobei die pharmazeutische Zusammensetzung insgesamt 150 bis 250 mg Furazidin, bevorzugt 180 bis 220 mg Furazidin und mehr bevorzugt 200 mg Furazidin, umfasst.

4. Pharmazeutische Zusammensetzung gemäß Anspruch 3, wobei die Komponente mit sofortiger Freisetzung a) 35 bis 55 mg Furazidin umfasst und die Komponente mit modifizierter Freisetzung b) 145 bis 165 mg Furazidin umfasst; und bevorzugt die Komponente mit sofortiger Freisetzung a) 45 bis 55 mg Furazidin umfasst und die Komponente mit modifizierter Freisetzung b) 145 bis 155 mg Furazidin umfasst; und mehr bevorzugt die Komponente mit sofortiger Freisetzung a) 50 mg Furazidin umfasst und die Komponente mit modifizierter Freisetzung b) 150 mg Furazidin umfasst.

5. Pharmazeutische Zusammensetzung gemäß einem der Ansprüche 1 bis 4, wobei die Komponente mit modifizierter Freisetzung b) 10 bis 40 Gew.-% des Mittels zur kontrollierten Freisetzung, bevorzugt 10 bis 35 Gew.-% und mehr bevorzugt 20 bis 30 Gew.-% des Mittels zur kontrollierter Freisetzung, bezogen auf das Gesamtgewicht der Komponente mit modifizierter Freisetzung b), umfasst.

6. Pharmazeutische Zusammensetzung gemäß einem der Ansprüche 1 bis 5, wobei die Komponente mit sofortiger Freisetzung a) als den mindestens einen Hilfsstoff mindestens einen von einem Füllstoff, einem Sprengmittel, einem Bindemittel, einem Gleitmittel, einem Fließregulierungsmittel und einer beliebigen Kombination davon umfasst,
bevorzugt die Komponente mit sofortiger Freisetzung a) 10 bis 50 Gew.-% eines Füllstoffs, 0,5 bis 6 Gew.-% eines Sprengmittels, 1,4 bis 1,7 Gew.-% eines Fließregulierungsmittels, 2 bis 4 Gew.-% eines Gleitmittels und optional 0,4 bis 5 Gew.-% eines Bindemittels, bezogen auf das Gesamtgewicht der Komponente mit sofortiger Freisetzung a), umfasst.

7. Pharmazeutische Zusammensetzung gemäß einem der Ansprüche 1 bis 6, wobei die Komponente mit modifizierter Freisetzung b) als den mindestens einen Hilfsstoff mindestens einen von einem Füllstoff, einem Sprengmittel, einem Bindemittel, einem Gleitmittel, einem Fließregulierungsmittel oder einer beliebigen Kombination davon umfasst,
bevorzugt die Komponente mit modifizierter Freisetzung b) 10 bis 40 Gew.-% des Mittels zur kontrollierten Freisetzung und 10 bis 45 Gew.-% eines Füllstoffs, 0,3 bis 5 Gew.-% eines Sprengmittels, 0,4 bis 5 Gew.-% eines Bindemittels, 0,5 bis 2 Gew.-% eines Gleitmittels und optional bis zu 1,3 Gew.-% eines Fließregulierungsmittels, bezogen auf das Gesamtgewicht der Komponente mit modifizierter Freisetzung b), umfasst.

8. Pharmazeutische Zusammensetzung gemäß einem der Ansprüche 1 bis 7, wobei die pharmazeutische Zusammensetzung eine in-vitro-Auflösungsrate von Furazidin von 10 bis 30% innerhalb von 1 h aufweist.

9. Pharmazeutische Zusammensetzung, wie in einem der Ansprüche 1 bis 8 definiert, zur Verwendung in einem Verfahren zur Behandlung einer Harnwegsinfektion.

10. Pharmazeutische Zusammensetzung zur Verwendung gemäß Anspruch 9, wobei die Harnwegsinfektion eine akute oder rezidivierende unkomplizierte Harnwegsinfektion ist.

11. Pharmazeutische Zusammensetzung zur Verwendung gemäß Anspruch 9 oder 10, wobei die pharmazeutische Zusammensetzung eine Einzeldosisform ist, die oral zweimal täglich und bevorzugt im Abstand von 11 bis 13 Stunden und mehr bevorzugt im Abstand von 12 Stunden verabreicht wird.

## Revendications

1. Composition pharmaceutique à libération prolongée pour une administration orale, comprenant :
a) un composant à libération immédiate, comprenant de la furazidine et un ou plusieurs excipients pharmaceutiquement acceptables ; et
b) un composant à libération modifiée, comprenant de la furazidine, un agent à libération contrôlée, et un ou plusieurs excipients pharmaceutiquement acceptables,
dans laquelle
la composition pharmaceutique est une forme posologique unitaire sous la forme d'un comprimé bicouche, dans lequel le composant à libération immédiate a) forme une première couche, le composant à libération modifiée b) forme une seconde couche, et la première couche recouvre au moins partiellement la surface de la seconde couche,
dans une matrice du composant à libération modifiée b), l'agent à libération contrôlée comprend au moins un polymère indépendant du pH, qui est au moins un sélectionné dans le groupe consistant en hydroxypropylméthylcellulose (HMPC), hydroxyéthylcellulose (HEC), hydroxypropylcellulose, gomme xanthane, carboxyméthylcellulose sodique, poly(oxyde d'éthylène) non ionique, et toute combinaison de ceux-ci,
le composant à libération immédiate présente un taux de dissolution in vitro d'au moins 80 % en poids de la furazidine en 45 minutes, sur la base de la teneur totale en furazidine du composant à libération immédiate,
le composant à libération modifiée présente un taux de dissolution in vitro de 5 à 15 % en poids de furazidine en 2 h, sur la base de la teneur totale en furazidine du composant à libération modifiée, et
la composition pharmaceutique à libération prolongée présente un taux de dissolution in vitro de furazidine de 10 à 40 % en poids en 1 h, de 40 à 65 % en poids en 6 h et supérieur à 80 % en poids en 14 h, sur la base de la teneur totale en furazidine de la forme posologique unitaire, et
les taux de dissolution étant déterminés en utilisant des paniers d'appareil « 1 » de la Pharmacopée européenne édition 10 dans un volume de 1000 ml de solution tamponnée au phosphate de pH = 6,8 avec l'ajout de CTAB (bromure d'hexadécyltriméthylammonium) à 2,5 % à 37,0 °C ± 0,5 °C et 100 tr/min.

2. Composition pharmaceutique selon la revendication 1, dans laquelle le composant à libération immédiate a) est contenu sous forme de granulés, et le composant à libération modifiée b) est contenu sous forme de granulés,
dans laquelle le composant à libération immédiate a) et/ou le composant à libération modifiée b) contiennent également facultativement une fraction extragranulaire comprenant un ou plusieurs d'un lubrifiant, d'une charge, d'un absorbant et d'un agent de glissement.

3. Composition pharmaceutique selon la revendication 1 ou 2, dans laquelle la composition pharmaceutique comprend, au total, 150 à 250 mg de furazidine, préférablement 180 à 220 mg de furazidine, et plus préférablement 200 mg de furazidine.

4. Composition pharmaceutique selon la revendication 3, dans laquelle le composant à libération immédiate a) comprend 35 à 55 mg de furazidine, et le composant à libération modifiée b) comprend 145 à 165 mg de furazidine ; et, préférablement, le composant à libération immédiate a) comprend 45 à 55 mg de furazidine, et le composant à libération modifiée b) comprend 145 à 155 mg de furazidine ; et, plus préférablement, le composant à libération immédiate a) comprend 50 mg de furazidine, et le composant à libération modifiée b) comprend 150 mg de furazidine.

5. Composition pharmaceutique selon l'une quelconque des revendications 1 à 4, dans laquelle le composant à libération modifiée b) comprend 10 à 40 % en poids de l'agent à libération contrôlée, préférablement 10 à 35 % en poids et plus préférablement 20 à 30 % en poids de l'agent à libération contrôlée, sur la base du poids total du composant à libération modifiée b).

6. Composition pharmaceutique selon l'une quelconque des revendications 1 à 5, dans laquelle le composant à libération immédiate a) comprend, en tant qu'au moins un excipient, au moins l'un d'une charge, d'un délitant, d'un liant, d'un lubrifiant, d'un agent de glissement et toute combinaison de ceux-ci,
préférablement, le composant à libération immédiate a) comprend 10 à 50 % en poids d'une charge, 0,5 à 6 % en poids d'un délitant, 1,4 à 1,7 % en poids d'un agent de glissement, 2 à 4 % en poids d'un lubrifiant, et, facultativement, 0,4 à 5 % en poids d'un liant, sur la base du poids total du composant à libération immédiate a).

7. Composition pharmaceutique selon l'une quelconque des revendications 1 à 6, dans laquelle le composant à libération modifiée b) comprend, en tant qu'au moins un excipient, au moins l'un d'une charge, d'un délitant, d'un liant, d'un lubrifiant, d'un agent de glissement et toute combinaison de ceux-ci,
préférablement, le composant à libération modifiée b) comprend 10 à 40 % en poids de l'agent à libération contrôlée et 10 à 45 % en poids d'une charge, 0,3 à 5 % en poids d'un délitant, 0,4 à 5 % en poids d'un liant, 0,5 à 2 % en poids d'un lubrifiant, et, facultativement, jusqu'à 1,3 % en poids d'un agent de glissement, sur la base du poids total du composant à libération modifiée b).

8. Composition pharmaceutique selon l'une quelconque des revendications 1 à 7, dans laquelle la composition pharmaceutique présente un taux de dissolution in vitro de furazidine de 10 à 30 % en 1 h.

9. Composition pharmaceutique selon l'une quelconque des revendications 1 à 8 pour une utilisation dans un procédé de traitement d'une infection des voies urinaires.

10. Composition pharmaceutique pour une utilisation selon la revendication 9, dans laquelle l'infection des voies urinaires est une infection non compliquée aiguë ou récurrente des voies urinaires.

11. Composition pharmaceutique pour une utilisation selon la revendication 9 ou 10, dans laquelle la composition pharmaceutique est une forme posologique unitaire, qui est administrée par voie orale deux fois par jour, et préférablement à une distance de 11 à 13 heures, et plus préférablement à une distance de 12 heures.
